# EUROPEAN PATENT APPLICATION

(11) **EP 4 729 000 A1**
(43) Date of publication of application: **22.04.2026**
(21) Application number: 24823499.9
(22) Date of filing: 17.06.2024
(51) Int. Cl.: A61B 17/122

(54) **CLIP UNIT**

(30) Priority: 16.06.2023 US 202363508588 P; 17.10.2023 US 202363590939 P
(71) Applicant: Olympus Medical Systems Corp., Tokyo 192-8507 (JP)
(72) Inventor: SHIRAI Jun, Hachioji-shi, Tokyo 192-8507 (JP)
(74) Representative: Schicker, Silvia
(86) International application number: PCT/JP2024/021944
(87) International publication number: WO 2024/257886

(57) **Abstract**

A clip unit includes a clip having a first arm and a second arm that are able to be opened and closed in an opening and closing direction, wherein the first arm has a first arm main body extending in a longitudinal direction along a longitudinal axis, a first claw that is provided at a distal end of the first arm main body and extends toward a closing side in the opening and closing direction, and a first anchor that is provided at the distal end of the first arm main body or the first claw and extends toward the closing side in the opening and closing direction, wherein at least a distal end of the first anchor is disposed on an opening side in the opening and closing direction with respect to the first claw, and wherein a gap is formed between the first claw and the first anchor in a width direction perpendicular to the longitudinal direction and the opening and closing direction.

## Description

### TECHNICAL FIELD

The present invention relates to a clip unit. Priority is claimed on United Sates Provisional Patent Application No. 63/508,588, filed June 16, 2023 and United Sates Provisional Patent Application No. 63/590,939, filed October 17, 2023, the contents of which are incorporated herein by reference.

### BACKGROUND ART

In endoscopic treatment, a clip unit that can ligate an excised portion after treatment and can stop bleeding or the like is used. The clip unit includes a clip that clamps the excised portion or the like, a pressing tube that houses the clip and locks the clip in a closed state, and the like. The clip unit is introduced into a treatment position by a clip introducing device that can be inserted into and passes through a channel of an endoscope.

Patent Document 1 describes a treatment tool that punctures living body tissue. The treatment tool described in Patent Document 1 can be placed inside a living body in a state in which the treatment tool has punctured living body tissue.

### Citation List

### Patent Document

Patent Document 1: United States Patent Application, Publication No. 2022/0378431

### SUMMARY OF INVENTION

### Technical Problem

However, the treatment tool described in Patent Document 1 is not necessarily an optimal treatment tool for performing treatment such as suturing an excised portion or a defective portion of tissue.

In view of the above circumstances, an object of the present invention is to provide a clip unit that is optimal for treatment of suturing living body tissue.

### Solution to Problem

In order to solve the above problems, the present invention proposes the following means.

According to a first aspect of the present invention, there is provided a clip unit including: a clip having a first arm and a second arm that are able to be opened and closed in an opening and closing direction; and a tubular member that is able to store at least a part of the clip, wherein the first arm has a first arm main body extending in a longitudinal direction along a longitudinal axis, a first claw that is provided at a distal end of the first arm main body and extends toward a closing side in the opening and closing direction, and a first anchor that is provided at the distal end of the first arm main body or the first claw and extends toward the closing side in the opening and closing direction, wherein at least a distal end of the first anchor is disposed on an opening side in the opening and closing direction with respect to the first claw, and wherein a gap is formed between the first claw and the first anchor in a width direction perpendicular to the longitudinal direction and the opening and closing direction. Advantageous Effects of Invention

A clip unit of the present invention can be suitably used for performing, for example, treatment such as suturing an excised portion or a defective portion.

### BRIEF DESCRIPTION OF DRAWINGS

[FIG. 1] A perspective view of a clip introducing device in a clip device according to a first embodiment.
[FIG. 2] A perspective view of a clip unit of the clip device.
[FIG. 3] A perspective view of the clip unit in which a pressing member is transparently shown.
[FIG. 4] A view showing a clip in an open state.
[FIG. 5] A view showing the clip in a closed state.
[FIG. 6] A view showing the clip in a closed state as viewed in a longitudinal direction.
[FIG. 7] A cross-sectional view of the pressing member in the longitudinal direction.
[FIG. 8] A perspective view of a pressing tube of the pressing member.
[FIG. 9] A perspective view of the pressing tube as viewed from a proximal end side.
[FIG. 10] A perspective view of a fastening member of the pressing tube.
[FIG. 11] A perspective view of a connecting member of the clip unit.
[FIG. 12] A view showing the clip unit loaded in the clip introducing device.
[FIG. 13] A view showing the clip unit introduced into a living body.
[FIG. 14] A view showing a first anchor and a second anchor that have punctured mucous membranes.
[FIG. 15] A view showing a first mucous membrane and a second mucous membrane which are pulled together.
[FIG. 16] A view showing the first anchor that has punctured the first mucous membrane.
[FIG. 17] A view showing the first mucous membrane pulled toward the second mucous membrane.
[FIG. 18] A view showing the clip unit with the clip closed.
[FIG. 19] A view showing the clip unit with the clip closed.
[FIG. 20] A view showing the clip unit with the clip locked.
[FIG. 21] A view showing the clip unit with the clip locked.
[FIG. 22] A view showing the clip unit with the clip separated.
[FIG. 23] A view showing the clip unit with the clip separated.
[FIG. 24] A view showing a clip in an open state in a clip unit according to a second embodiment.
[FIG. 25] A view showing the clip in a closed state.
[FIG. 26] A view showing the clip in a closed state as viewed in a longitudinal direction.
[FIG. 27] A view showing a clip in an open state in a clip unit according to a third embodiment.
[FIG. 28] A view showing the clip in a closed state.
[FIG. 29] A view showing the clip in a closed state as viewed in a longitudinal direction.
[FIG. 30] A view showing a modification example of a first anchor and a modification example of a second anchor of the clip.
[FIG. 31] A view showing another modification example of the first anchor and another modification example of the second anchor of the clip.
[FIG. 32] A view showing another modification example of the first anchor and another modification example of the second anchor of the clip.
[FIG. 33] A view showing a clip in an open state in a clip unit according to a fourth embodiment.
[FIG. 34] A view showing the clip in a closed state.
[FIG. 35] A view showing the clip in a closed state as viewed in a longitudinal direction.
[FIG. 36] A view showing a clip in an open state in a clip unit according to a fifth embodiment.
[FIG. 37] A view showing the clip in a closed state.
[FIG. 38] A view showing the clip in a closed state as viewed in a longitudinal direction.
[FIG. 39] A view showing a modification example of a first anchor and a modification example of a second anchor of the clip.
[FIG. 40] A view showing the modification example of the first anchor and the modification example of the second anchor of the clip.
[FIG. 41] A view showing another modification example of the first anchor and another modification example of the second anchor.
[FIG. 42] A view showing the modification example of the first anchor and the modification example of the second anchor.
[FIG. 43] A view showing a clip in an open state in a clip unit according to a sixth embodiment.
[FIG. 44] A view showing the clip in a closed state.
[FIG. 45] A view showing the clip in a closed state as viewed in a longitudinal direction.
[FIG. 46] A view showing a clip in an open state in a clip unit according to a seventh embodiment.
[FIG. 47] A view showing the clip in a closed state.
[FIG. 48] A view showing the clip in a closed state as viewed in a longitudinal direction.
[FIG. 49] A view showing a modification example of a first anchor and a modification example of a second anchor of the clip.
[FIG. 50] A view showing the modification example of the first anchor and the modification example of the second anchor of the clip.
[FIG. 51] A view showing a clip in an open state in a clip unit according to an eighth embodiment.
[FIG. 52] A view showing the clip in a closed state.
[FIG. 53] A view showing the clip in a closed state as viewed in a longitudinal direction.
[FIG. 54] A view showing a clip in an open state in a clip unit according to a ninth embodiment.
[FIG. 55] A view showing the clip in a closed state.
[FIG. 56] A view showing the clip in a closed state as viewed in a longitudinal direction.
[FIG. 57] A view showing a manufacturing method of a first arm of the first embodiment.
[FIG. 58] A view showing a manufacturing method of a first arm of the ninth embodiment.

### DESCRIPTION OF EMBODIMENTS

### (First embodiment)

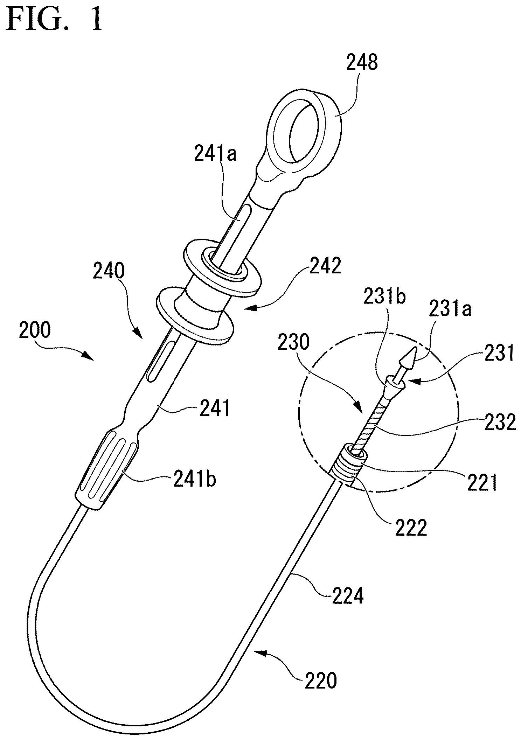

A clip device 300 according to a first embodiment of the present invention will be described with reference to FIGS. 1 to 23.

### [Clip device 300]

The clip device (clip system) 300 includes a clip introducing device (applicator) 200 and a clip unit 1. The clip unit 1 is loaded into the clip introducing device 200.

### [Clip introducing device 200]

FIG. 1 is a perspective view of the clip introducing device 200.

The clip introducing device (applicator) 200 includes a sheath 220, an operation wire 230, and an operation section 240. The clip introducing device 200 is inserted into and passes through, for example, a treatment tool insertion and passage channel of an endoscope and is used in combination with the endoscope. For this reason, the sheath 220 is formed to be sufficiently longer than the length of the treatment instrument insertion and passage channel of the endoscope. The sheath 220 is flexible and curves in accordance with the curvature of an insertion section of the endoscope.

The sheath 220 includes a distal end tip 221, a distal end side coil 222, and a proximal side coil 224 and is formed in the shape of a long and thin tube as a whole. The distal end side coil 222 is disposed on a distal end portion side of the sheath 220. The distal end tip 221 is disposed at a distal end portion of the distal end side coil 222.

As shown in FIG. 1, the operation wire (power transmission unit) 230 includes an arrowhead hook (connection section) 231 connected to the clip unit 1 and a wire 232 for operating the arrowhead hook 231.

The arrowhead hook 231 includes a substantially conical engaging portion 231a that engages with the clip unit 1, and a wire connecting portion 231b provided at a proximal end of the engaging portion 231a. The arrowhead hook 231 is formed of, for example, a metal material such as a stainless steel material.

The wire 232 is inserted into and passes through the sheath 220 so as to be able to advance and retract. The distal end portion of the wire 232 is fixed to a proximal end of the wire connecting portion 231b through welding, for example.

As shown in FIG. 1, the operation section 240 includes an operation section main body 241, a slider 242, and a thumb ring 248. The operation section main body 241, the slider 242, and the thumb ring 248 are formed of, for example, a resin material in an injection molding manner. The operation section main body 241 includes a slit portion 241a and a rotating grip 241b at a distal end side. The slit portion 241a supports the slider 242 such that the slider 242 can advance and retract.

The slider 242 is attached to the operation section main body 241 so as to be able to advance and retract in a longitudinal axis direction, and the proximal end of the wire 232 is attached to the slider 242. As the slider 242 advances and retracts along the operation section main body 241, the wire 232 advances and retracts relative to the sheath 220, and the arrowhead hook 231 advances and retracts.

The thumb ring 248 is attached to a proximal end of the operation section main body 241 so as to be rotatable around a longitudinal axis of the operation section main body 241.

### [Clip unit 1]

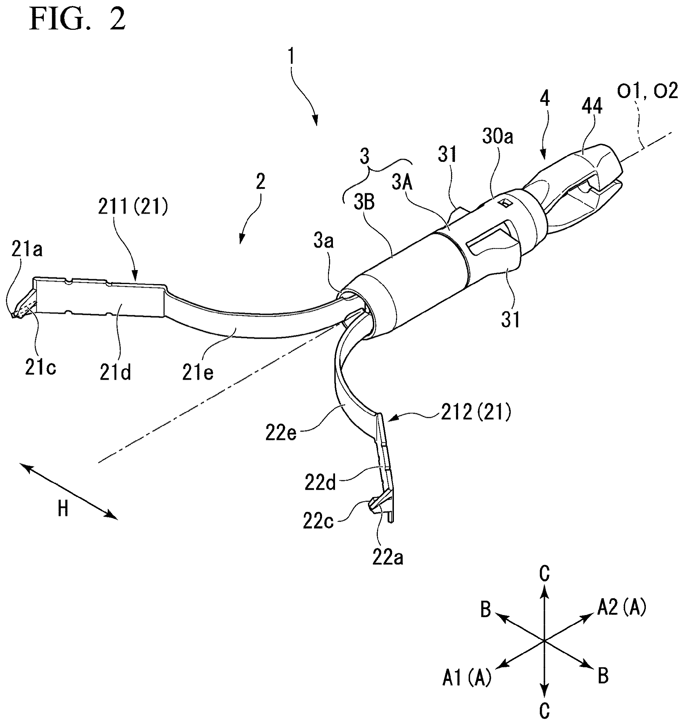

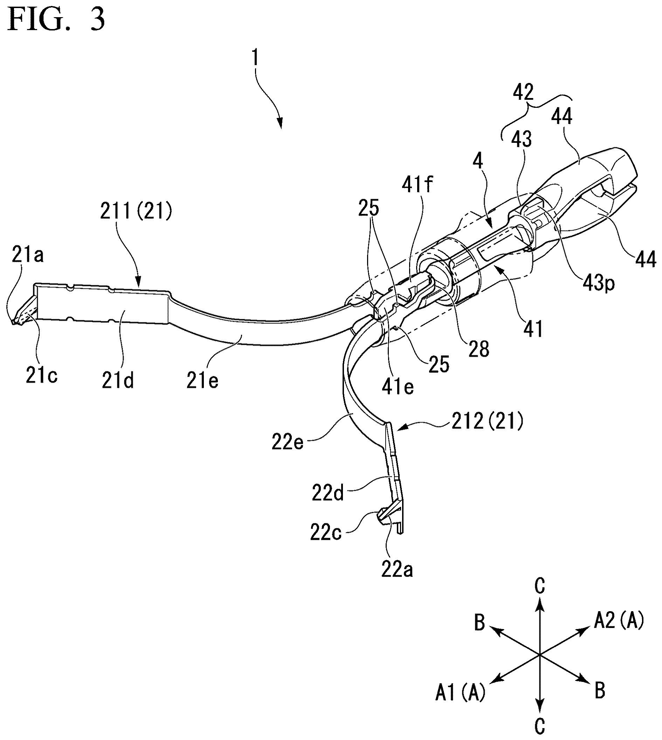

FIG. 2 is a perspective view of the clip unit 1. FIG. 3 is a perspective view of the clip unit 1 in which a pressing member 3 is transparently shown. The clip unit 1 includes a clip 2, a pressing member 3, and a connecting member 4.

In the following description, in a longitudinal direction A of the clip unit 1, a side of the clip 2 will be referred to as a distal end side (distal side) A1 of the clip unit 1, and a side of the connecting member 4 will be referred to as a proximal end side (proximal side) A2 of the clip unit 1. In addition, a direction perpendicular to the longitudinal direction A is referred to as a "left-right direction B," and a direction perpendicular to the longitudinal direction A and the left-right direction B is referred to as an "up-down direction C."

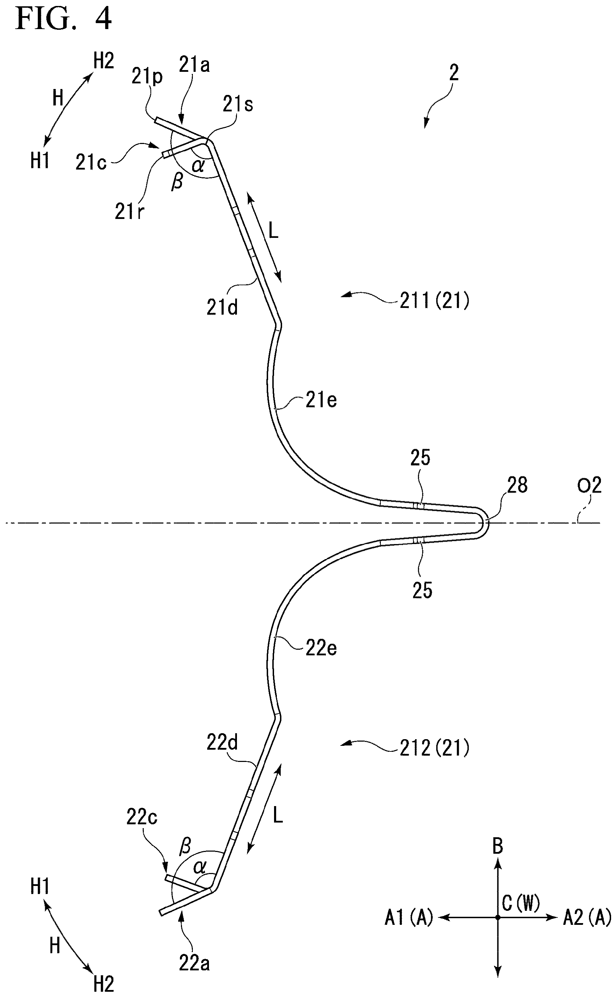

FIG. 4 is a view showing the clip 2 in an open state.

The clip (clip arm) 2 is formed by bending a metal plate material at a central portion. The clip 2 has a pair of arms 21 that can be opened and closed toward the distal end side A1, and a proximal end portion 28 that connects the pair of arms 21. The proximal end portion 28 is provided with an engaging portion 25. The proximal end side A2 of the clip 2 is inserted into an internal space 38 of the pressing member 3.

The pair of arms 21 have a first arm 211 and a second arm 212 that can be opened and closed in an opening and closing direction H. The first arm 211 and the second arm 212 are disposed on both sides of a central axis O1 of the clip unit 1 in the longitudinal direction A. The clip 2 may have three or more arms.

In the opening and closing direction H, a direction in which one arm approaches the other arm is defined as a "closing side H1." In the opening and closing direction H, a direction in which one arm moves away from the other arm is defined as an "opening side H2." A direction in which the pair of arms 21 face each other is referred to as an "inner side," and a side opposite to the inner side is referred to as an "outer side."

The first arm 211 has a first anchor 21a, a first claw 21c, a first arm main body 21d, and a first sliding portion 21e.

The second arm 212 has a second anchor 22a, a second claw 22c, a second arm main body 22d, and a second sliding portion 22e. The second arm 212 has a similar shape to the first arm 211, and thus only the different portions will be described below.

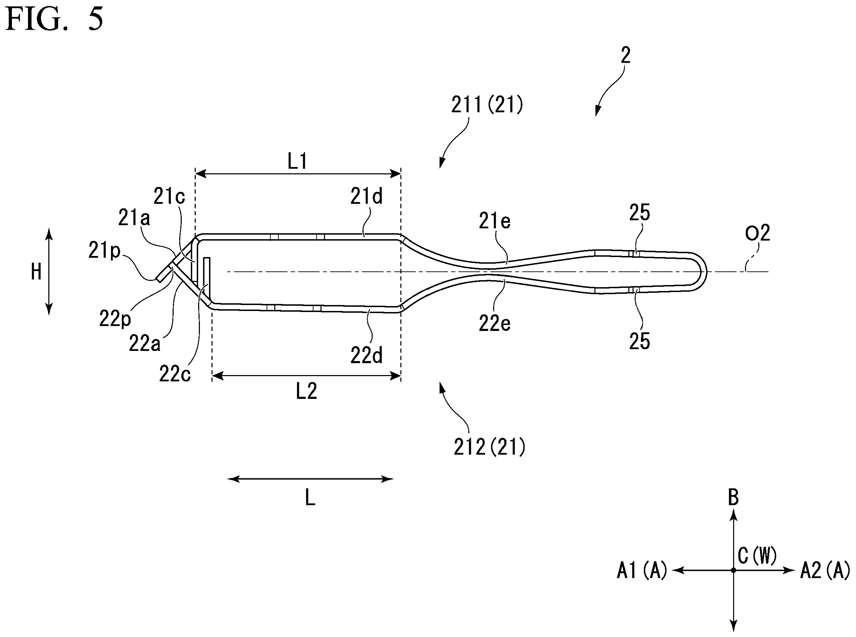

FIG. 5 is a view showing the clip 2 in a closed state.

The first arm main body 21d is formed in a flat plate shape extending in a longitudinal direction L. The first arm main body 21d is a portion that mainly grasps tissue. When the clip 2 is in a closed state, the longitudinal direction L substantially coincides with the longitudinal direction A. A direction perpendicular to the longitudinal direction L and the opening and closing direction H is defined as a "width direction W."

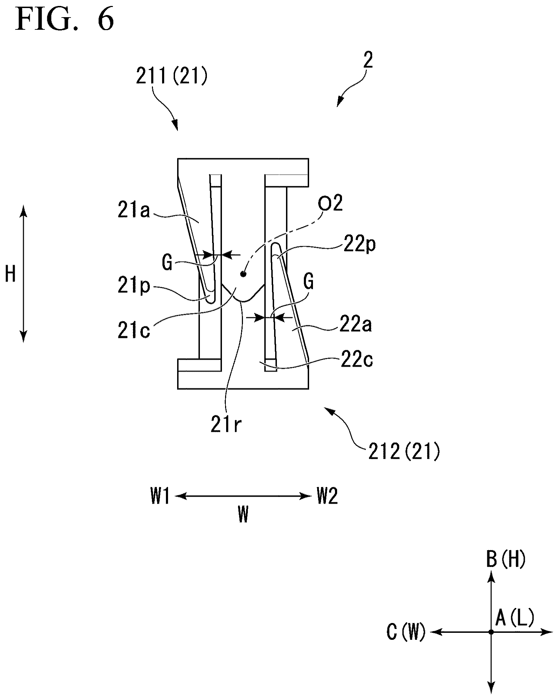

FIG. 6 is a view showing the clip 2 in a closed state as viewed in the longitudinal direction L.

The first claw 21c and the first anchor 21a are spaced apart from each other in the width direction W. A gap G is formed between the first claw 21c and the first anchor 21a in the width direction W. For this reason, the first anchor 21a can easily puncture a mucous membrane.

The first claw 21c is provided at a distal end 21s of the first arm main body 21d and extends toward the closing side H1 in the opening and closing direction H. The first claw 21c is disposed in the center in the width direction W. A distal end 21r of the first claw 21c is formed with an uneven surface, which makes it easy to grasp the mucous membrane.

The first anchor 21a is provided at the distal end 21s of the first arm main body 21d and extends toward the closing side H1 in the opening and closing direction H. The first anchor 21a is disposed on one side W1 in the width direction W. A distal end 21p of the first anchor 21a is formed in a sharp tapered shape, and thus it is possible to easily puncture the mucous membrane. The first anchor 21a is formed in the shape of a right triangle when viewed in the longitudinal direction L. The first anchor 21a extends inward in the width direction W, and the distal end 21p is less likely to come into contact with the channel of the endoscope.

The first anchor 21a is disposed on the opening side H2 in the opening and closing direction H with respect to the first claw 21c. The first claw 21c and the first anchor 21a extend from the same portion (distal end 21s) when viewed in the width direction W as shown in FIG. 4. The first claw 21c and the first anchor 21a do not have to extend from the same portion when viewed in the width direction W. It is sufficient that at least the distal end 21p of the first anchor 21a is disposed on the opening side H2 in the opening and closing direction H with respect to the first claw 21c.

As shown in FIG. 6, the length of the first anchor 21a in the opening and closing direction H is smaller than the length of the first claw 21c in the opening and closing direction H. The length of the first anchor 21a in the width direction W is smaller than the length of the first claw 21c in the width direction W.

A first angle β formed between the first anchor 21a and the first arm main body 21d is greater than a second angle α formed between the first claw 21c and the first arm main body 21d. For example, the first angle β is preferably greater than or equal to 90 degrees and less than or equal to 135 degrees. This is because when the clip 2 in an open state approaches the tissue from the front, the distal end 21p of the first anchor 21a can easily come into contact with the tissue.

When the clip 2 is in an open state, the distal end 21p of the first anchor 21a is preferably disposed on the distal end side A1 with respect to the distal end 21r of the first claw 21c. This is because when the clip 2 in an open state approaches the tissue from the front, the distal end 21p of the first anchor 21a comes into contact with the tissue first.

As shown in FIG. 6, when the clip 2 is in a closed state, the first claw 21c and the second claw 22c overlap with each other when viewed in the longitudinal direction L, and the first claw 21c is disposed on the distal end side A1 with respect to the second claw 22c. The second claw 22c may have a notch to prevent interference with the first claw 21c.

As shown in FIG. 6, the first anchor 21a is disposed on one side W1 in the width direction W. The second anchor 22a is disposed on the other side W2 in the width direction W. When the clip 2 is in a closed state, the first anchor 21a and the second anchor 22a do not overlap with each other and are disposed to be spaced apart from each other when viewed in the longitudinal direction L, and are disposed rotationally symmetrical with respect to a central axis O2 in the longitudinal direction A of the clip 2. When the clip 2 is in a closed state, the first anchor 21a and the second anchor 22a are disposed to overlap with each other at an overlapping position in the opening and closing direction H, and can puncture the mucous membrane without interfering with each other.

As shown in FIG. 5, when the clip 2 is in a closed state, the distal end 21p of the first anchor 21a and the distal end 22p of the second anchor 22a are disposed at positions shifted in the longitudinal direction A, and thus it is possible to easily puncture the mucous membrane.

The first arm 211 and the second arm 212 are formed in an asymmetrical shape with respect to the central axis O2. As shown in FIG. 5, a length L1 of the first arm main body 21d in the longitudinal direction L is greater than a length L2 of the second arm main body 22d in the longitudinal direction L.

The first sliding portion 21e is a portion that is elastically deformed when the first arm 211 is drawn into the pressing member 3. A distal end of the first sliding portion 21e is connected to the first arm main body 21d. A proximal end of the first sliding portion 21e is connected to the base end portion 28.

The engaging portion 25 is a member that can engage with a fastening member 32 of the pressing member 3. The distal end side A1 of the engaging portion 25 is formed as an inclined surface at an obtuse angle relative to the longitudinal direction A, and the proximal end side A2 thereof is formed as an inclined surface at an acute angle relative to the longitudinal direction A.

The proximal end portion 28 connects the first arm 211 and the second arm 212. The first arm 211 and the second arm 212 connected by the proximal end portion 28 are provided so as to be able to open and close toward the distal end side A1. The proximal end portion 28 is bent into a U-shape and is connected to a hook 41 f of the connecting member 4. The proximal end portion 28 is biased such that the pair of arms 21 are in an open state. For this reason, the pair of arms 21 of the clip 2 have a self-expanding force in the opening and closing direction H.

As shown in FIG. 3, the proximal end portion 28 and the hook 41f of the connecting member 4 are connected by inserting the hook 41f of the connecting member 4 into the proximal end portion 28 which is formed in a U-shape. When the proximal end portion 28 is connected to the connecting member 4, the opening and closing direction H of the pair of arms 21 substantially coincides with the left-right direction B.

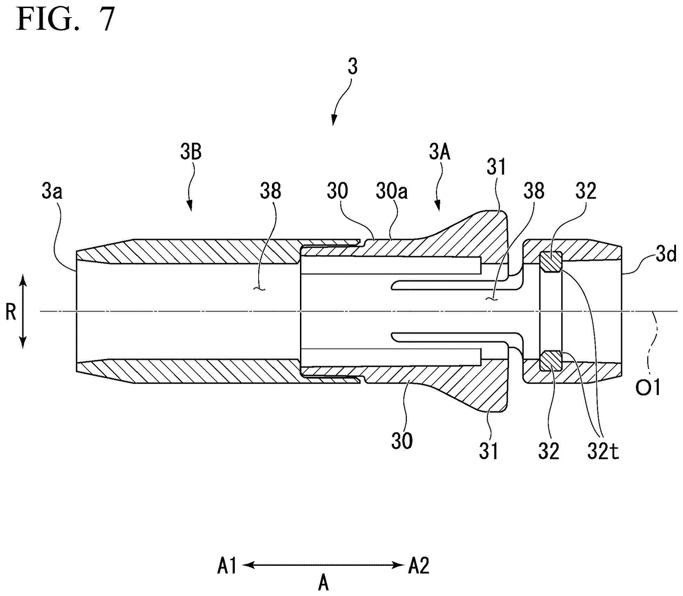

FIG. 7 is a cross-sectional view of the pressing member 3 in the longitudinal direction A.

The pressing member (tubular member, holder) 3 is a cylindrical member capable of storing at least a part of the clip 2 therein. The pressing member 3 has the internal space 38 through which the clip 2 advances and retracts in the longitudinal direction A. The pressing member 3 can fix the clip 2 drawn into the internal space 38 in a closed state. The pressing member 3 has a pressing tube 3A provided on the proximal end side A2 and a pressing pipe 3B provided on the distal end side A1.

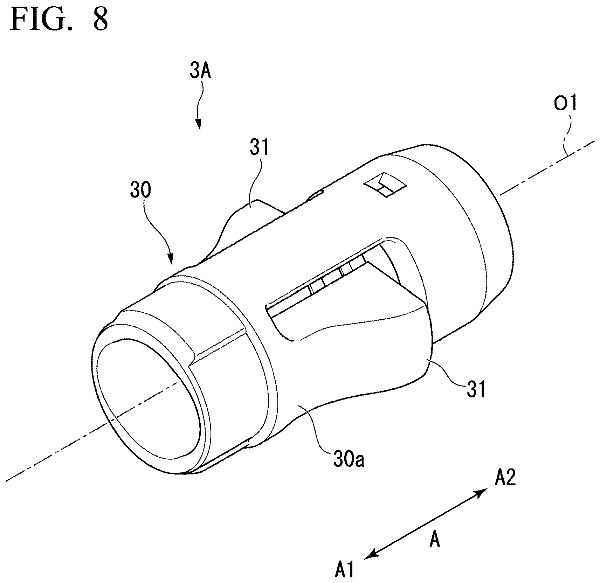

FIG. 8 is a perspective view of the pressing tube 3A.

The pressing tube (second tubular member) 3Ahas a pressing tube main body 30 formed in a cylindrical shape, a projecting and retracting wing 31, and a fastening member 32.

The pressing tube main body 30 is formed by injection molding a material softer than the clip 2, for example, a thermoplastic resin with moderate elasticity, such as polyphthalamide (PPA), polyamide (PA), polyether ether ketone (PEEK), or a liquid crystal polymer (LCP). The pressing tube main body 30 may be made of a metal instead of a thermoplastic resin.

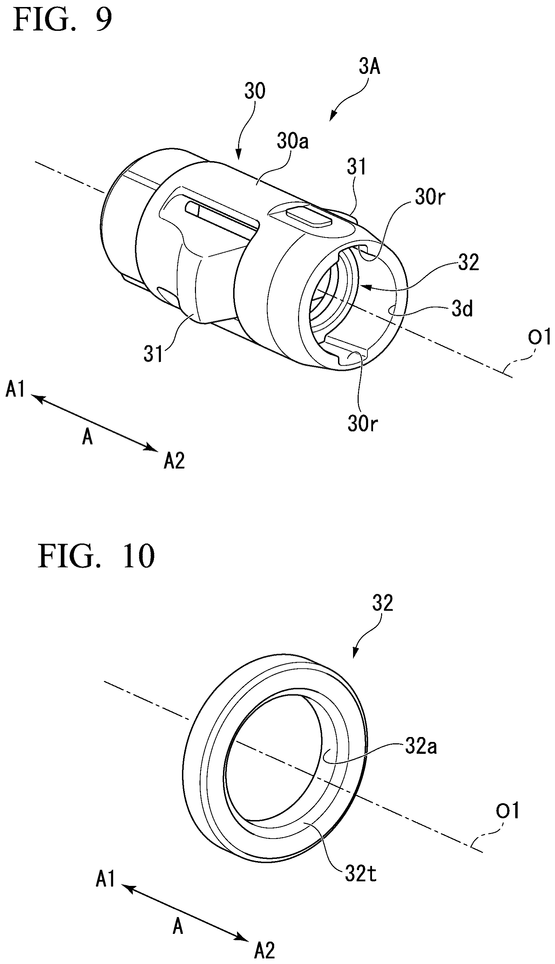

FIG. 9 is a perspective view of the pressing tube 3A as viewed from the proximal end side A2.

An engaging concave portion 30r that engages with an engaging convex portion 43p of the connecting member 4 is formed in a proximal end opening 3d of the pressing tube main body 30. The engaging concave portions 30r are provided on both sides of the central axis O1.

The projecting and retracting wings 31 are a pair of protrusions that project and retract with respect to an outer circumferential surface 30a of the pressing tube main body 30. The projecting and retracting wings 31 are provided on both sides of the central axis O1. The projecting and retracting wing 31 has a projected state, in which the projecting and retracting wing 31 protrudes to an outer side in a radial direction R with respect to the outer circumferential surface 30a, as a basic orientation. When the projecting and retracting wing 31 receives a force directed from an outer side to an inner side in the radial direction R, the projecting and retracting wing 31 becomes a retracted state in which the projecting and retracting wing 31 retracts with respect to the outer circumferential surface 30a. When the above force is released, the projecting and retracting wing 31 returns from the retracted state to the projected state.

The fastening member 32 is a ring-shaped member provided in the internal space 38 of the pressing tube 3A. The fastening member 32 is made of a metal. The fastening member 32 may be formed to be harder than the pressing tube main body 30, and may be formed of, for example, a thermoplastic resin instead of a metal.

The fastening member 32 is disposed such that a central axis of the fastening member 32 coincides with the central axis O1. The fastening member 32 is disposed on the proximal end side A2 with respect to the projecting and retracting wing 31. The fastening member 32 is incorporated into the pressing tube main body 30 through, for example, insert molding. The fastening member 32 is disposed at a position where the fastening member 32 protrudes to the inner side in the radial direction R from the inner circumferential surface of the pressing tube main body 30.

FIG. 10 is a perspective view of the fastening member 32.

The fastening member 32 is formed with a tapered surface 32t on the proximal end side A2, an inner diameter of which decreases from the proximal end side A2 toward the distal end side A1. Since the fastening member 32 has the tapered surface 32t, the connecting member 4 can be easily inserted into the fastening member 32 from the proximal end side A2.

The pressing pipe (first tubular member) 3B is a cylindrical member made of a metal. The pressing pipe 3B is press-fitted into a distal end portion of the pressing tube 3A. The pressing tube 3A and the pressing pipe 3B may be connected through heat welding, adhesion, or screw fastening.

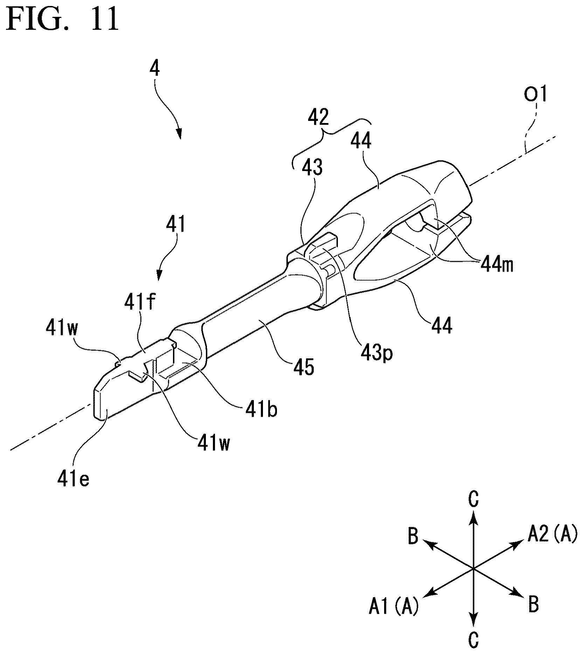

FIG. 11 is a perspective view of the connecting member 4.

The connecting member 4 is detachably connected to the proximal end portion 28 of the clip 2. In addition, the connecting member 4 is detachably connected to the arrowhead hook 231 that is inserted into and passes through the inside of the sheath 220. That is, the connecting member 4 connects the clip 2 and the arrowhead hook 231. The connecting member 4 includes a distal end connecting portion 41 provided at a distal end thereof, a proximal end connecting portion 42 provided at a proximal end thereof, and a rod-shaped portion 45 that connects the distal end connecting portion 41 and the proximal end connecting portion 42.

The distal end connecting portion 41 has the hook 41f at a distal end portion thereof, a breaking portion 41b provided on the proximal end side A2 of the hook 41f, a distal end expanding portion 41e, and a first protrusion 41w. The distal end connecting portion 41 can be inserted into and pass through the internal space 38 of the pressing member 3 while connected to the clip 2.

The hook 41f is a hook that extends in the up-down direction C perpendicular to the central axis O1 and is formed in a substantially cylindrical rod shape. The hook 41f extends from a "lower side" to an "upper side" in the up-down direction C. The proximal end portion 28 of the clip 2 is hooked onto the hook 41f from above.

The breaking portion 41b breaks when a tensile breaking force is applied to the hook 41f as the proximal end portion 28 is pulled toward the proximal end side A2. The breaking portion 41b may have any mechanism for releasing the connection between the proximal end portion 28 of the clip 2 and the hook 41f of the connecting member 4. For example, the breaking portion 41b may have a mechanism that releases the connection between the proximal end portion 28 and the hook 41f by deforming (plastically deforming or elastically deforming) without breaking.

The distal end expanding portion 41e is a member that is provided on the hook 41f and protrudes toward the distal end side A1. The distal end expanding portion 41e has a tapered shape that narrows toward the distal end side A1 in both the up-down direction C and the left-right direction B.

The first protrusion 41w is a member that is provided on the hook 41f and protrudes in the left-right direction B. The first protrusions 41w are a pair of wingshaped protrusions extending on both sides in the left-right direction B at the upper end of the hook 41f extending upward in the up-down direction C. The first protrusions 41w have shapes that are symmetrical with respect to the central axis O1.

The proximal end connecting portion 42 is an engaging portion with which the arrowhead hook 231 of the clip introducing device 200 is engaged (connected). The proximal end connecting portion 42 has a connecting portion main body 43 and a connecting arm 44.

The connecting portion main body 43 is provided at a proximal end of the rod-shaped portion 45. The connecting portion main body 43 is formed with an engaging convex portion 43p that protrudes in the up-down direction. The engaging convex portion 43p engages with the engaging concave portion 30r provided in the proximal end opening of the pressing member 3, and thus relative rotation between the pressing member 3 and the connecting member 4 in a circumferential direction with respect to the longitudinal direction A is restricted.

The connecting arm (pair of legs) 44 is provided at the proximal end of the connecting portion main body 43 and is branched into a bifurcated shape. The connecting arm 44 is elastically deformable in the up-down direction C with respect to the connecting portion main body 43 and is openable and closable with respect to the connecting portion main body 43. The connecting arm 44 is formed with a notch 44m for gripping and accommodating the engaging portion 231a of the arrowhead hook 231. The notch 44m is formed in a shape that fits closely to an outer circumferential surface of the engaging portion 231a of the arrowhead hook 231. The connecting arm 44 can be connected to the arrowhead hook 231 of the clip introducing device 200 in an open state and can maintain the connection with the arrowhead hook 231 of the clip introducing device 200 in a closed state.

The rod-shaped portion 45 is a substantially rod-shaped member extending in the longitudinal direction A. The rod-shaped portion 45 is provided between the distal end connecting portion 41 and the proximal end connecting portion 42 and connects the distal end connecting portion 41 and the proximal end connecting portion 42. The rod-shaped portion 45 can be inserted into and pass through the internal space 38 of the pressing member 3.

### [Motion and operation of clip unit 1]

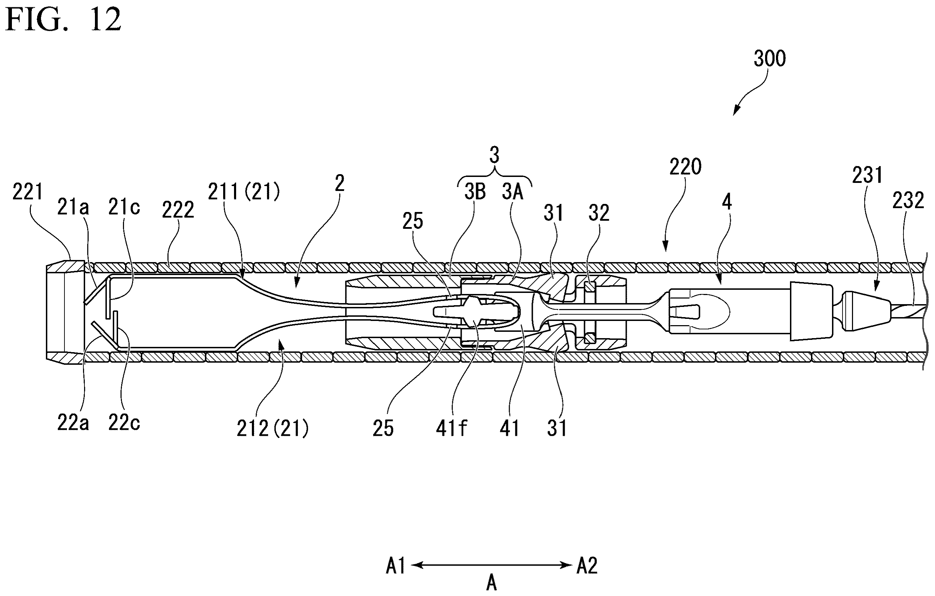

Next, the motion and operation of the clip unit 1 will be described with reference to FIGS. 12 to 23.

FIG. 12 is a view showing the clip unit 1 loaded in the clip introducing device 200.

The clip unit 1 is loaded into the clip introducing device 200 using a cartridge or the like. The connecting member 4 of the clip unit 1 loaded in the clip introducing device 200 is connected to an arrowhead hook 231 that is inserted into and passes through the inside of the sheath 220. The projecting and retracting wings 31 are pressed against an inner circumferential surface of the sheath 220 and are in the retracted state.

The pair of arms 21 of the loaded clip unit 1 are pressed against the inner circumferential surface of the sheath 220 and are in a closed state. The engaging portion 25 is located on the distal end side A1 with respect to the fastening member 32, and the pair of arms 21 are not locked in a closed state.

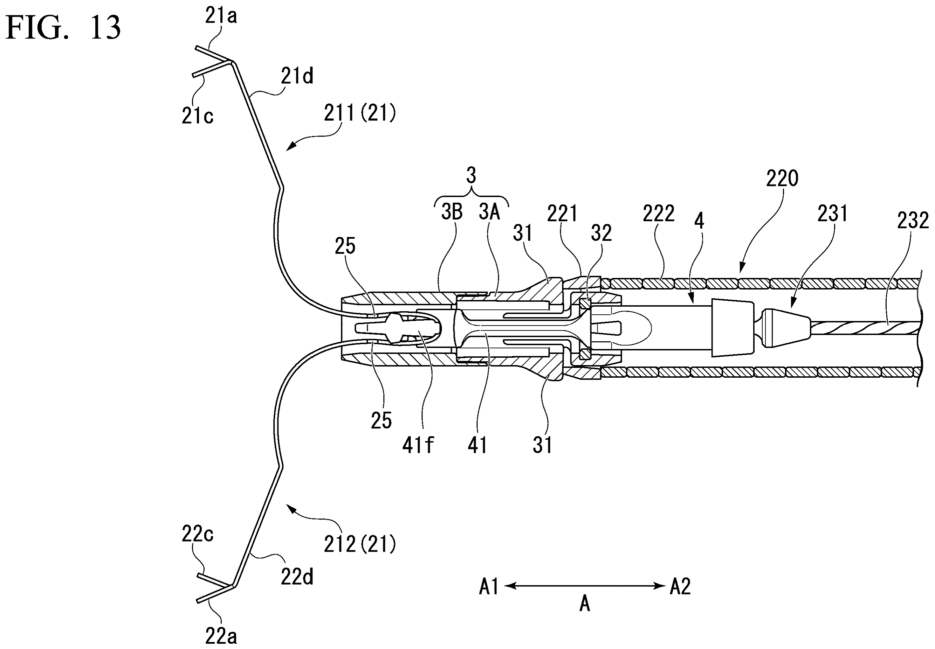

FIG. 13 is a view showing the clip unit 1 introduced into a living body.

A surgeon introduces the clip unit 1 loaded in the sheath 220 into the living body via the channel of the endoscope. Next, the surgeon advances the slider 242 along the operation section main body 241 to advance the arrowhead hook 231. The surgeon advances the clip unit 1 until the projecting and retracting wings 31 come out of the sheath 220. When the projecting and retracting wings 31 come out of the sheath 220, the projecting and retracting wings 31 return from the retracted state to the projected state, which is the basic orientation.

When the distal end sides of the pair of arms 21 come out of the sheath 220, the self-expanding force of the pair of arms 21 acts as a restoring force, and the clip 2 returns to the open state while moving to the distal end side with respect to the pressing member 3. Even in a case in which the pair of arms 21 return to the open state and protrude from the pressing member 3 to the maximum, the engaging portion 25 is disposed in the internal space 38 of the pressing member 3.

The surgeon moves the endoscope or the sheath 220 to bring the clip 2 of the clip unit 1 closer to a defective portion D, which is a treatment region. In the following description, the mucous membranes that are located at a periphery surrounding the defective portion D and are disposed opposite to each other with the defective portion D interposed therebetween will be referred to as a "first mucous membrane M1" and a "second mucous membrane M2."

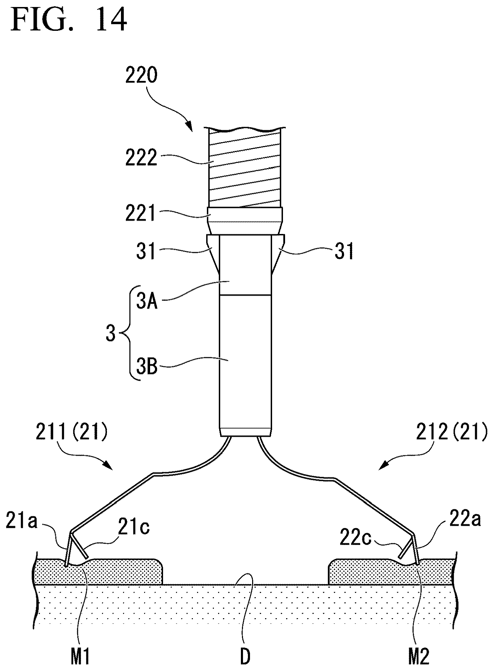

FIG. 14 is a view showing the first anchor 21a and the second anchor 22a that have punctured the mucous membranes. In a case in which the defective portion D is small, the surgeon punctures the first mucous membrane M1 with the first anchor 21a and punctures the second mucous membrane M2 with the second anchor 22a. The first anchor 21a and the second anchor 22a extend toward the closing side H1 in the opening and closing direction H, but are disposed on the opening side H2 in the opening and closing direction H with respect to the first claw 21c and the second claw 22c. For this reason, when the clip 2 in an open state is disposed in front of the mucous membrane, the first anchor 21a and the second anchor 22a are disposed at a position that make it easy to puncture the mucous membrane.

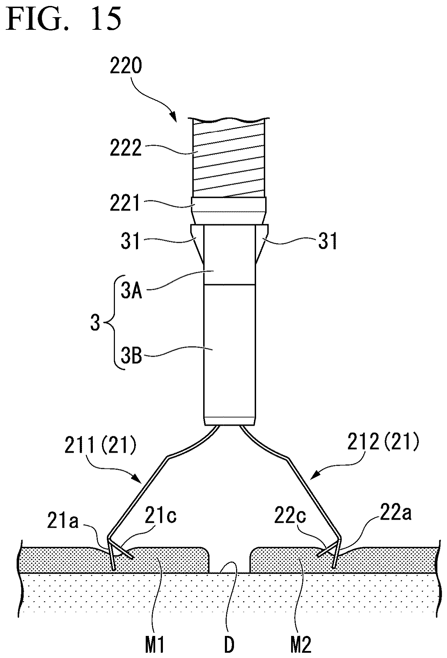

FIG. 15 a view showing the first mucous membrane M1 and the second mucous membrane M2 which are pulled together.

The surgeon can pull together the first mucous membrane M1 and the second mucous membrane M2 by pulling together the first arm 211 of which the first anchor 21a has punctured the first mucous membrane M1 and the second arm 212 of which the second anchor 22a has punctured the second mucous membrane M2.

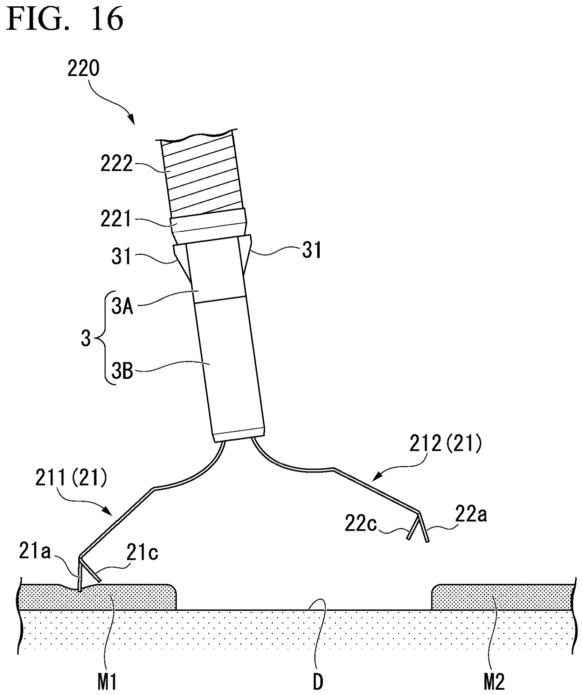

FIG. 16 is a view showing the first anchor 21a that has punctured the first mucous membrane M1.

In a case in which the defective portion D is large, the surgeon first punctures the first mucous membrane M1 with the first anchor 21a. For the reasons described above, the first anchor 21a is disposed at a position that makes it easy to puncture the mucous membrane.

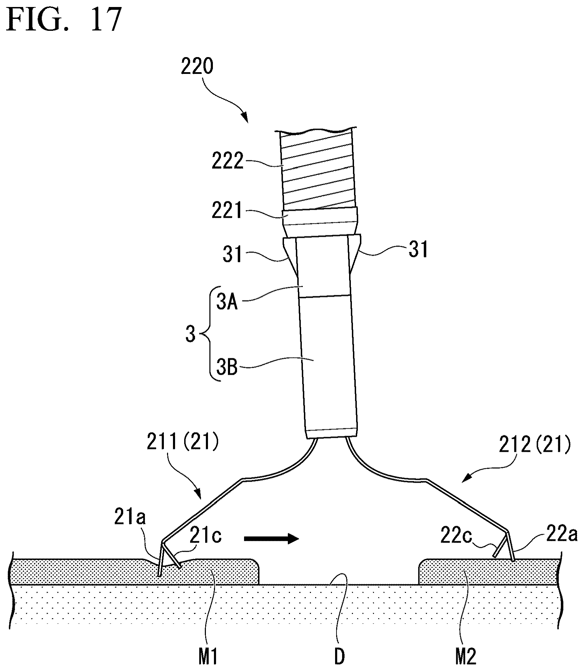

FIG. 17 is a view showing the first mucous membrane M1 pulled toward the second mucous membrane M2.

The surgeon pulls the first mucous membrane M1 punctured by the first anchor 21a toward the second mucous membrane M2 side. Since the first mucous membrane M1 is firmly punctured by the first anchor 21a, the surgeon can easily pull the first mucosa M1 toward the second mucous membrane M2 side.

The surgeon punctures the second mucous membrane M2 with the second anchor 22a. For the reasons described above, the first anchor 21a is disposed at a position that makes it easy to puncture the mucous membrane.

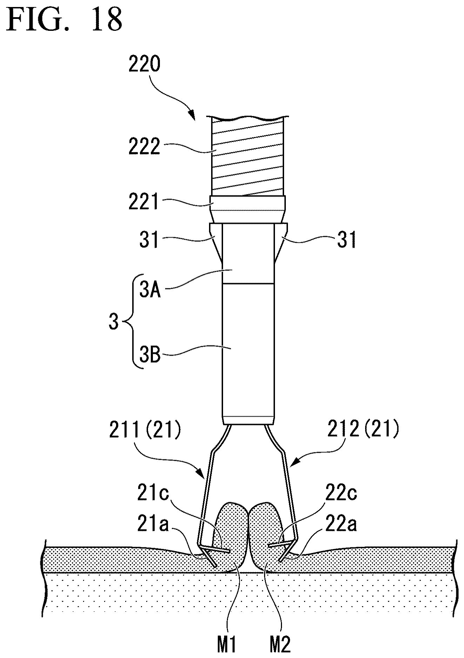

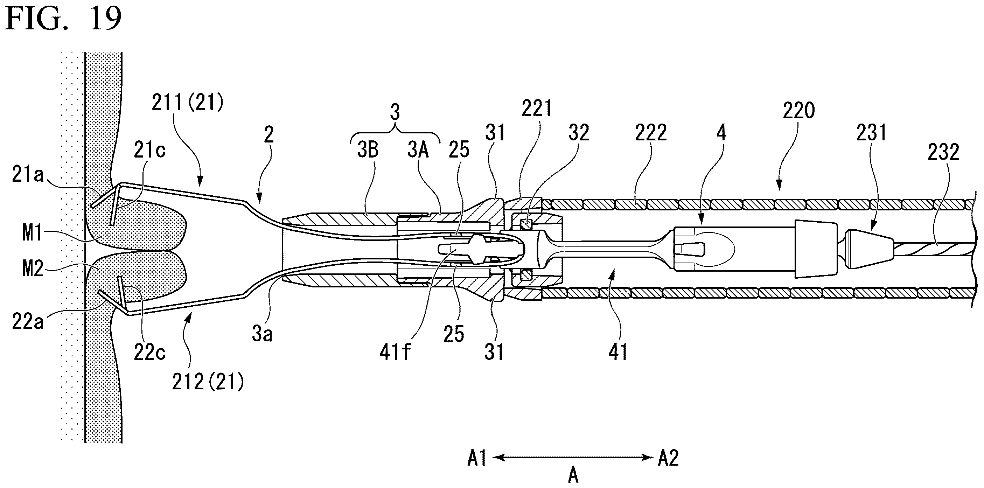

FIGS. 18 and 19 are views showing the clip unit 1 with the pair of arms 21 closed.

The surgeon retracts the slider 242 along the operation section main body 241 to retract the arrowhead hook 231. The connecting member 4 connected to the arrowhead hook 231 pulls the clip 2. The pair of arms 21 having a self-expanding force are pulled toward the proximal end side A2, thereby pushing a distal end opening 3a of the pressing member 3 toward the proximal end side A2. The projecting and retracting wings 31 in the projected state are engaged with the sheath 220 and thus are not drawn into the sheath 220. For this reason, the clip 2 pulled by the connecting member 4 is drawn into the pressing member 3.

The first mucous membrane M1 and the second mucous membrane M2 swell and are firmly pressed and grasped by the first claw 21c and the second claw 22c.

When the proximal end portion 28 of the clip 2 is pulled toward the proximal end side A2 of the pressing member 3 by the connecting member 4, the pair of arms 21 are drawn into the pressing member 3, and the pair of arms 21 are gradually closed. When a pulling force on the proximal end portion 28 is released in this state, the self-expanding force of the pair of arms 21 acts as a restoring force, and the clip 2 returns to the open state while moving toward the distal end side A1. The surgeon can return the pair of arms 21 to the open state to re-grasp the tissue.

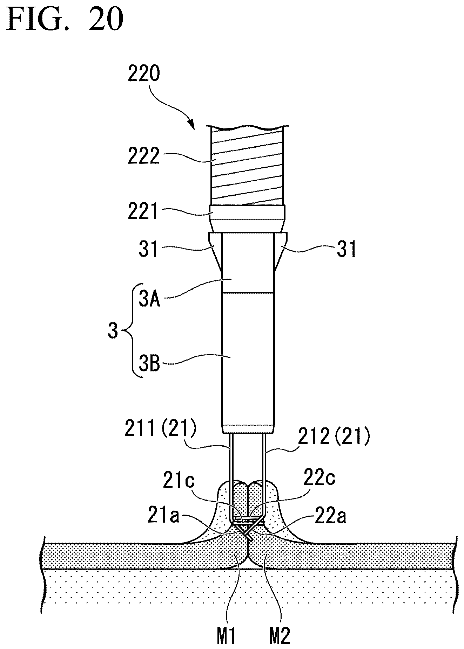

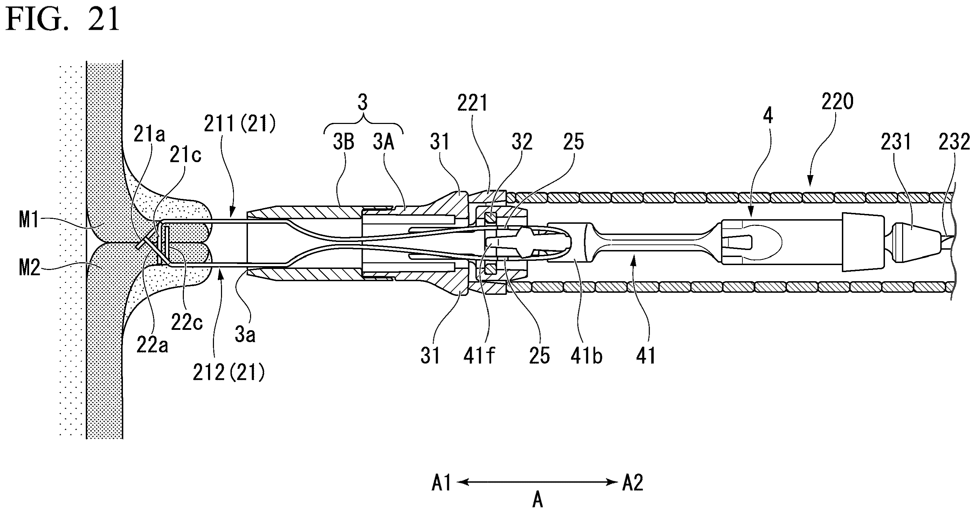

FIGS. 20 and 21 are views showing the clip unit 1 with the clip 2 locked.

As the proximal end portion 28 is further pulled toward the proximal end side A2 of the pressing member 3, the engaging portion 25 is drawn toward the proximal end side A2 with respect to the fastening member 32. The proximal end side A2 of the engaging portion 25 is formed as the inclined surface at an obtuse angle, and thus the engaging portion 25 can be easily drawn to the proximal end side with respect to the fastening member 32. On the other hand, the distal end side A1 of the engaging portion 25 is formed as the inclined surface at an acute angle, and thus when the engaging portion 25 is drawn to the proximal end side A2 with respect to the fastening member 32, the engaging portion 25 and the fastening member 32 engage with each other. As a result, the movement of the clip 2 toward the distal end side A1 with respect to the pressing member 3 is restricted, and the pair of arms 21 are locked in a closed state. Once the pair of arms 21 are locked in the closed state, the pair of arms 21 cannot return to the open state.

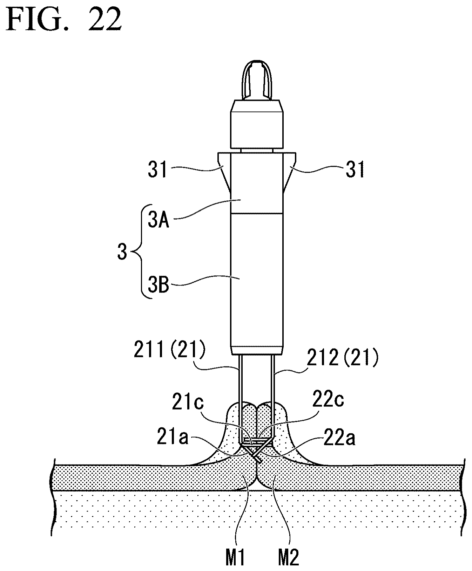

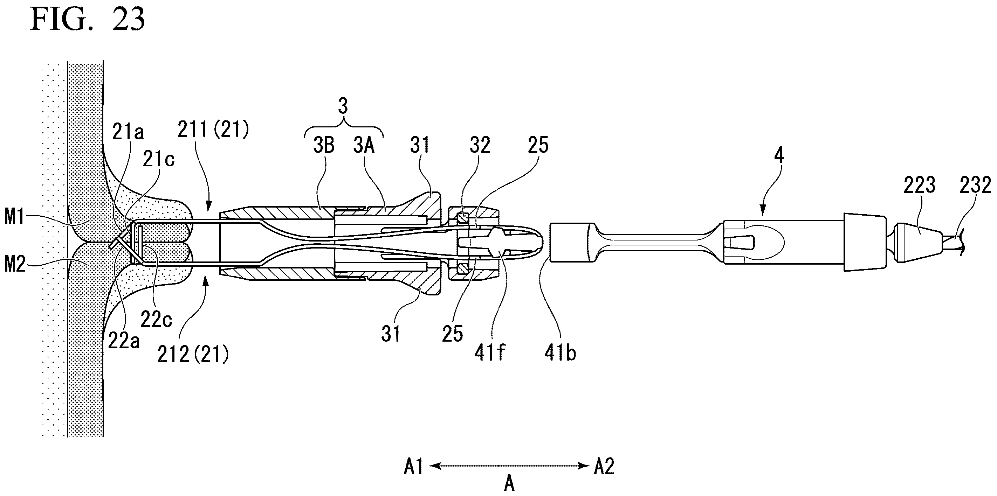

FIGS. 22 and 23 are views showing the clip unit 1 with the clip 2 separated.

The surgeon further pulls the clip 2. A tensile breaking force is applied to the hook 41f, and the breaking portion 41b breaks. The breaking strength of the breaking portion 41b is lower than the breaking strength of the connecting portion main body 43. For this reason, the breaking portion 41b breaks, not the connecting portion main body 43. The surgeon retracts the sheath 220 and leaves the clip 2 and the pressing member 3 in the living body in a state in which the clip 2 ligates the first mucous membrane M1 and the second mucous membrane M2.

The surgeon reloads a new clip unit 1 into the clip introducing device 200 using a cartridge or the like.

According to the clip unit 1 of the present embodiment, it is possible to suitably carry out the treatment of suturing the defective portion D. Even in a case in which the defective portion D is large, it is possible to pull the mucous membrane punctured by the first anchor 21a or the second anchor 22a, and it is possible to press and grasp the mucous membrane by the first claw 21c and the second claw 22c.

In the above, the first embodiment of the present invention has been described in detail with reference to the drawings, but the specific configuration is not limited to the embodiment, and a design change and the like within a range not departing from the gist of the present invention are also included. In addition, the constituent elements shown in the embodiment described above and a modification example which will be shown below can be appropriately combined and configured.

### (Second embodiment)

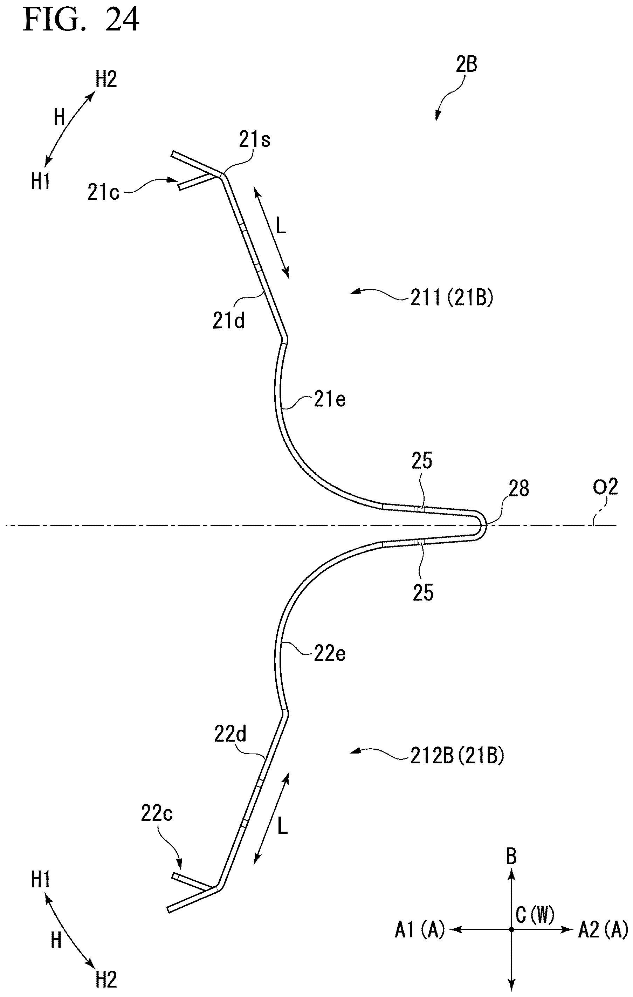

A clip unit 1B according to a second embodiment of the present invention will be described with reference to FIGS. 24 to 26. In the following description, the same constituent elements as those already described are designated by the same reference signs, and duplicate description will be omitted.

The clip unit 1B includes a clip 2B, the pressing member 3, and the connecting member 4. The clip unit 1B is loaded into the clip introducing device 200.

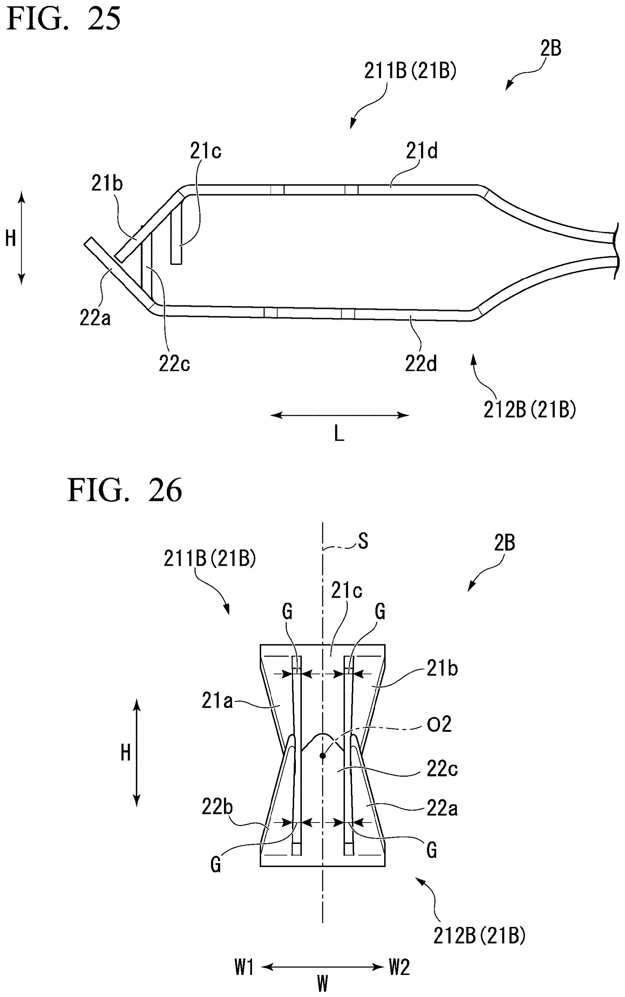

FIG. 24 is a view showing the clip 2B in an open state. FIG. 25 is a view showing the clip 2B in a closed state. The clip (clip arm) 2B is formed by bending a metal plate material at a central portion. The clip 2B has a pair of arms 21B that can be opened and closed toward the distal end side A1, and a proximal end portion 28 that connects the pair of arms 21B.

The pair of arms 21B have a first arm 211B and a second arm 212B that can be opened and closed in the opening and closing direction H. The first arm 211B and the second arm 212B are disposed on both sides of the central axis O1 of the clip unit 1B in the longitudinal direction A.

The first arm 211B has a first anchor 21a, a first auxiliary anchor 21b, a first claw 21c, a first arm main body 21d, and a first sliding portion 21e.

The second arm 212B has a second anchor 22a, a second auxiliary anchor 22b, a second claw 22c, a second arm main body 22d, and a second sliding portion 22e. The second arm 212B has a similar shape to the first arm 211B, and thus only the different portions will be described below.

FIG. 26 is a view showing the clip 2B in a closed state as viewed in the longitudinal direction L.

The first claw 21c and the first auxiliary anchor 21b are spaced apart from each other in the width direction W. A gap G is formed between the first claw 21c and the first auxiliary anchor 21b in the width direction W. For this reason, the first auxiliary anchor 21b can easily puncture the mucous membrane.

As shown in FIG. 26, the first anchor 21a is disposed on one side W1 in the width direction W. The first auxiliary anchor 21b is disposed on the other side W2 in the width direction W. The first anchor 21a and the first auxiliary anchor 21b are provided on both sides of the first claw 21c in the width direction W. The first anchor 21a and the first auxiliary anchor 21b are disposed symmetrically with respect to a central plane S. Here, the central plane S is a plane that extends in the longitudinal direction L and the opening and closing direction H and passes through a central axis O2.

The first auxiliary anchor 21b is disposed on an opening side H2 in the opening and closing direction H with respect to the first claw 21c.

When the clip 2B is in a closed state, the first claw 21c and the second claw 22c overlap with each other when viewed in the longitudinal direction L, and the second claw 22c is disposed on the distal end side with respect to the first claw 21c.

According to the clip unit 1B of the present embodiment, it is possible to suitably carry out the treatment of suturing a defective portion D. The clip unit 1B can suitably puncture the mucous membrane by using the first auxiliary anchor 21b in addition to the first anchor 21a.

In the above, the second embodiment of the present invention has been described in detail with reference to the drawings, but the specific configuration is not limited to the embodiment, and a design change and the like within a range not departing from the gist of the present invention are also included. In addition, the constituent elements shown in the embodiment described above and a modification example which will be shown below can be appropriately combined and configured.

### (Third embodiment)

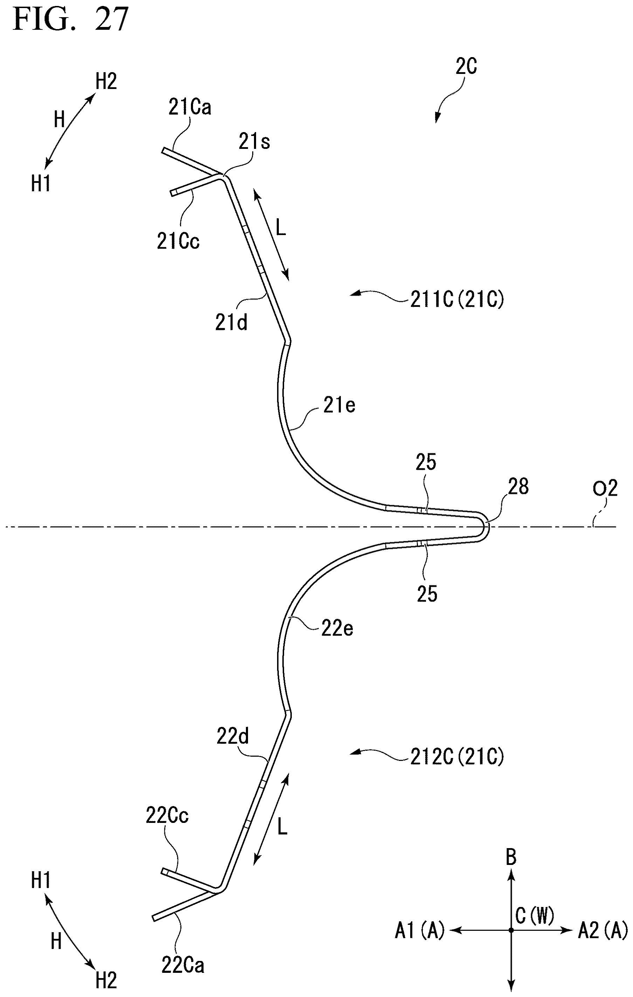

A clip unit 1C according to a third embodiment of the present invention will be described with reference to FIGS. 27 to 29. In the following description, the same constituent elements as those already described are designated by the same reference signs, and duplicate description will be omitted.

The clip unit 1C includes a clip 2C, the pressing member 3, and the connecting member 4. The clip unit 1C is loaded into the clip introducing device 200.

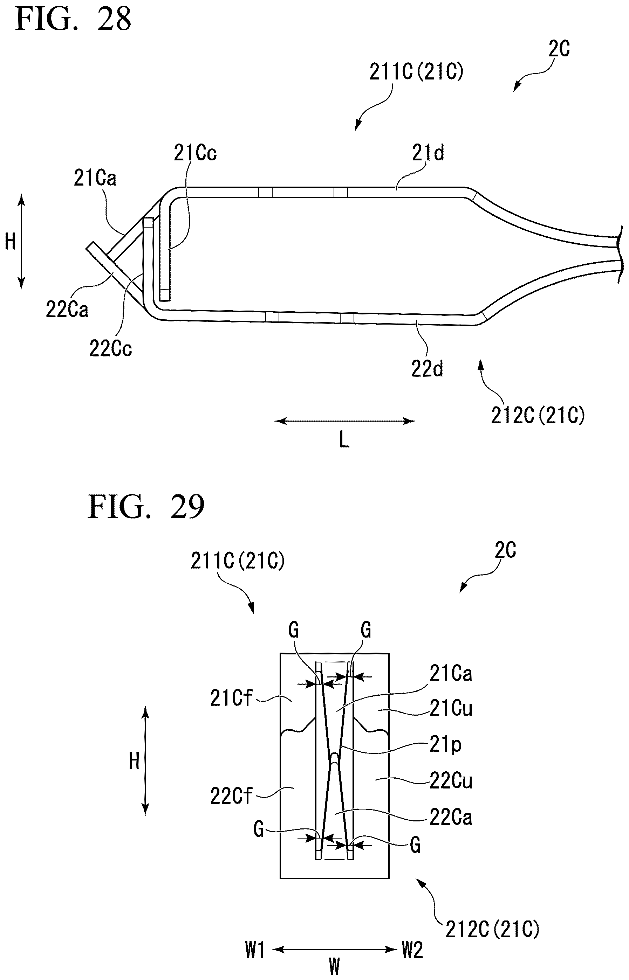

FIG. 27 is a view showing the clip 2C in an open state. FIG. 28 is a view showing the clip 2C in a closed state. The clip (clip arm) 2C is formed by bending a metal plate material at a central portion. The clip 2C has a pair of arms 21C that can be opened and closed toward the distal end side A1, and a proximal end portion 28 that connects the pair of arms 21C.

The pair of arms 21C have a first arm 211C and a second arm 212C that can be opened and closed in the opening and closing direction H. The first arm 211C and the second arm 212C are disposed on both sides of the central axis O1 of the clip unit 1C in the longitudinal direction A.

The first arm 211C has a first anchor 21Ca, a first claw 21Cc, a first arm main body 21d, and a first sliding portion 21e. The first claw 21Cc has a first upper claw 21Cu and a first lower claw 21Cf.

The second arm 212C has a second anchor 22Ca, a second claw 22Cc, a second arm main body 22d, and a second sliding portion 22e. The second claw 22Cc has a second upper claw 22Cu and a second lower claw 22Cf. The second arm 212C has a similar shape to the first arm 211C, and thus only the different portions will be described below.

FIG. 29 is a view showing the clip 2C in a closed state as viewed in the longitudinal direction L.

The first upper claw 21Cu and the first anchor 21Ca are spaced apart from each other in the width direction W. A gap G is formed between the first upper claw 21Cu and the first anchor 21Ca in the width direction W. The first lower claw 21Cf and the first anchor 21Ca are spaced apart from each other in the width direction W. A gap G is formed between the first lower claw 21Cf and the first anchor 21Ca in the width direction W. For this reason, the first anchor 21Ca can easily puncture the mucous membrane.

The first claw 21Cc (first upper claw 21Cu and first lower claw 21Cf) is provided at a distal end 21s of the first arm main body 21d and extends toward the closing side H1 in the opening and closing direction H. The first claw 21Cc (first upper claw 21Cu and the first lower claw 21Cf) is disposed on both sides of the first anchor 21Ca in the width direction W.

The first anchor 21Ca is provided at the distal end 21s of the first arm main body 21d and extends toward the closing side H1 in the opening and closing direction H. The first anchor 21Ca is disposed at the center in the width direction W. A distal end 21p of the first anchor 21Ca is formed in a sharp tapered shape, and thus it is possible to easily puncture the mucous membrane. The first anchor 21Ca is formed in an isosceles triangle shape when viewed in the longitudinal direction L.

The first anchor 21Ca is disposed on the opening side H2 in the opening and closing direction H with respect to the first claw 21Cc (first upper claw 21Cu and the first lower claw 21Cf).

When the clip 2C is in a closed state, the first anchor 21Ca and the second anchor 22Ca overlap with each other when viewed in the longitudinal direction L. The first anchor 21Ca and the second anchor 22Ca are disposed to overlap with each other at an overlapping position in the opening and closing direction H, and thus it is possible to easily puncture the mucous membrane.

According to the clip unit 1C of the present embodiment, it is possible to suitably carry out the treatment of suturing a defective portion D. Since the first anchor 21Ca or the second anchor 22Ca is located in the center in the width direction W, the first anchor 21Ca or the second anchor 22Ca can easily puncture the mucous membrane regardless of the angle of the clip 2C.

In the above, the third embodiment of the present invention has been described in detail with reference to the drawings, but the specific configuration is not limited to the embodiment, and a design change and the like within a range not departing from the gist of the present invention are also included. In addition, the constituent elements shown in the embodiment described above and a modification example which will be shown below can be appropriately combined and configured.

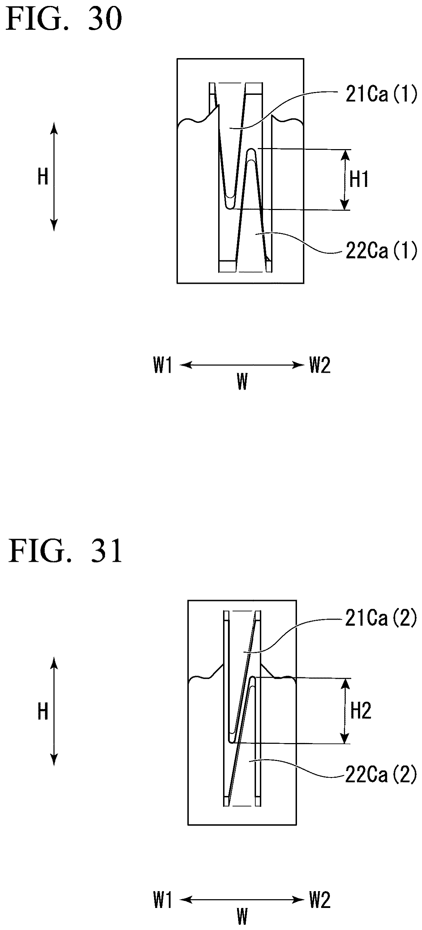

FIG. 30 is a view showing a first anchor 21Ca(1) which is a modification example of the first anchor 21Ca, and a second anchor 22Ca(1) which is a modification example of the second anchor 22Ca. When the clip 2C is in a closed state, the first anchor 21Ca(1) and the second anchor 22Ca(1) do not overlap with each other when viewed in the longitudinal direction L, but are disposed to overlap with each other at an overlapping position in the opening and closing direction H. The first anchor 21Ca(1) and second anchor 22Ca(1) have a longer length in the opening and closing direction H than the first anchor 21Ca and the second anchor 22Ca, and thus it is possible to easily puncture the mucous membrane.

FIG. 31 is a view showing a first anchor 21Ca(2) which is a modification example of the first anchor 21 Ca, and a second anchor 22Ca(2) which is a modification example of the second anchor 22Ca. When the clip 2C is in a closed state, the first anchor 21Ca(2) and the second anchor 22Ca(2) do not overlap with each other when viewed in the longitudinal direction L, but are disposed to overlap with each other at an overlapping position in the opening and closing direction H. The first anchor 21Ca(2) and the second anchor 22Ca(2) are formed in the shape of a right triangle when viewed in the longitudinal direction L. For this reason, a length H2 over which the first anchor 21Ca(2) and the second anchor 22Ca(2) overlap with each other in the opening and closing direction H is longer than a length H1 over which the first anchor 21Ca(1) and the second anchor 22Ca(1) overlap with each other in the opening and closing direction H.

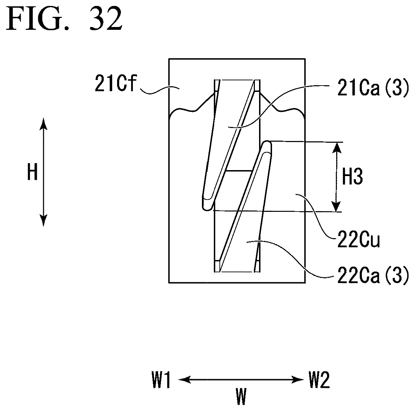

FIG. 32 is a view showing a first anchor 21Ca(3) which is a modification example of the first anchor 21Ca, and a second anchor 22Ca(3) which is a modification example of the second anchor 22Ca. When viewed in the longitudinal direction L, the distal end portion of the first anchor 21Ca(3) and the first lower claw 21Cf overlap with each other. When viewed in the longitudinal direction L, the distal end portion of the second anchor 22Ca(3) and the second upper claw 22Cu overlap with each other.

### (Fourth embodiment)

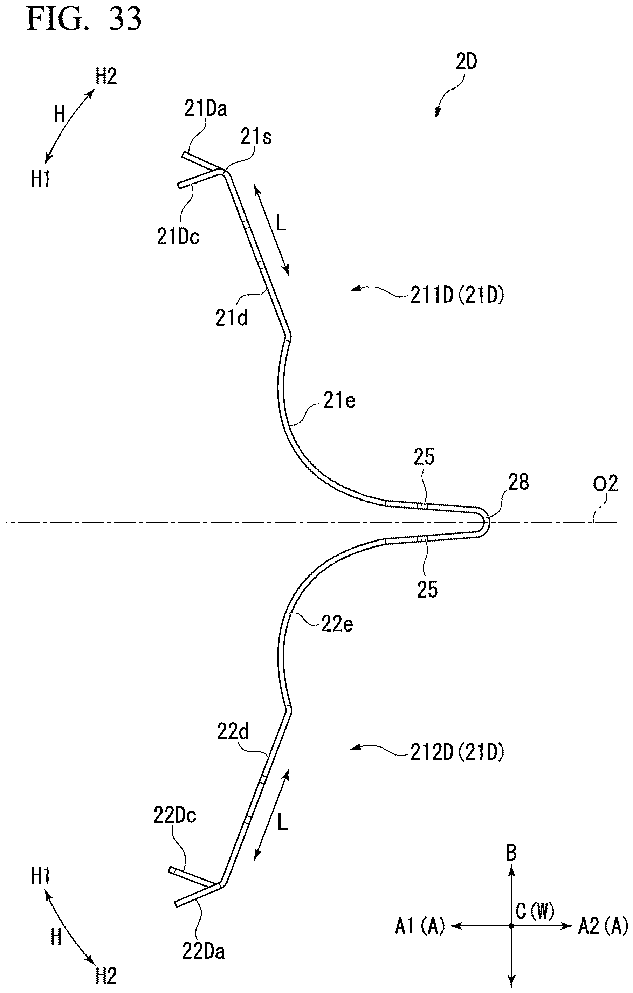

A clip unit 1D according to a fourth embodiment of the present invention will be described with reference to FIGS. 33 to 35. In the following description, the same constituent elements as those already described are designated by the same reference signs, and duplicate description will be omitted.

The clip unit 1D includes a clip 2D, the pressing member 3, and the connecting member 4. The clip unit 1D is loaded into the clip introducing device 200.

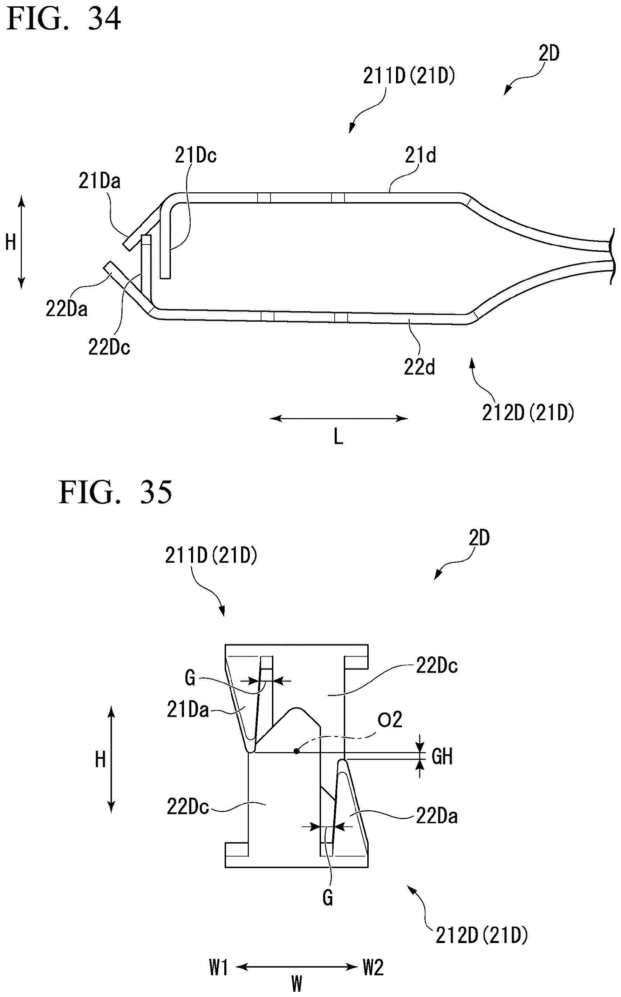

FIG. 33 is a view showing the clip 2D in an open state. FIG. 34 is a view showing the clip 2D in a closed state. The clip (clip arm) 2D is formed by bending a metal plate material at a central portion. The clip 2D has a pair of arms 21D that can be opened and closed toward the distal end side A1, and a proximal end portion 28 that connects the pair of arms 21D.

The pair of arms 21D have a first arm 211D and a second arm 212D that can be opened and closed in the opening and closing direction H. The first arm 211D and the second arm 212D are disposed on both sides of the central axis O1 of the clip unit 1D in the longitudinal direction A.

The first arm 211D has a first anchor 21Da, a first claw 21Dc, a first arm main body 21d, and a first sliding portion 21e.

The second arm 212D has a second anchor 22Da, a second claw 22Dc, a second arm main body 22d, and a second sliding portion 22e. The second arm 212D has a similar shape to the first arm 211D, and thus only the different portions will be described below.

FIG. 35 is a view showing the clip 2D in a closed state as viewed in the longitudinal direction L.

The first claw 21Dc and the first anchor 21Da are spaced apart from each other in the width direction W. A gap G is formed between the first claw 21Dc and the first anchor 21Da in the width direction W. For this reason, the first anchor 21Da can easily puncture the mucous membrane.

The first anchor 21Da is disposed on the opening side H2 in the opening and closing direction H with respect to the first claw 21 Dc.

When the clip 2D is in a closed state, the first anchor 21Da and the second anchor 22Da do not overlap with each other and are disposed to be spaced apart from each other when viewed in the longitudinal direction L, and are disposed rotationally symmetrical with respect to a central axis O2. The first anchor 21Da and the second anchor 22Da are disposed not to overlap with each other in the opening and closing direction H. A gap GH is formed between the first anchor 21Da and the second anchor 22Da in the opening and closing direction H. For this reason, the first anchor 21Da and the second anchor 22Da are shorter than those in other embodiments, and thus, can puncture the mucous membrane without bending.

According to the clip unit 1D of the present embodiment, it is possible to suitably carry out the treatment of suturing a defective portion D. Even in a case in which the defective portion D is large, it is possible to pull the mucous membrane punctured by the first anchor 21a or the second anchor 22a, and it is possible to press and grasp the mucous membrane by the first claw 21c and the second claw 22c.

In the above, the fourth embodiment of the present invention has been described in detail with reference to the drawings, but the specific configuration is not limited to the embodiment, and a design change and the like within a range not departing from the gist of the present invention are also included. In addition, the constituent elements shown in the embodiment described above and a modification example which will be shown below can be appropriately combined and configured.

### (Fifth embodiment)

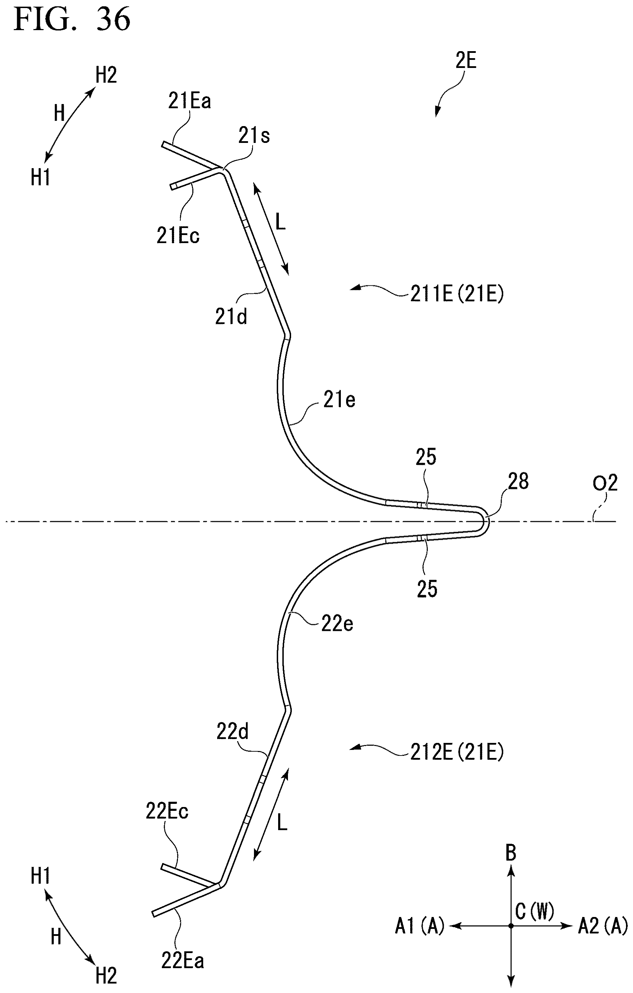

A clip unit 1E according to a fifth embodiment of the present invention will be described with reference to FIGS. 36 to 38. In the following description, the same constituent elements as those already described are designated by the same reference signs, and duplicate description will be omitted.

The clip unit 1E includes a clip 2E, the pressing member 3, and the connecting member 4. The clip unit 1E is loaded into the clip introducing device 200.

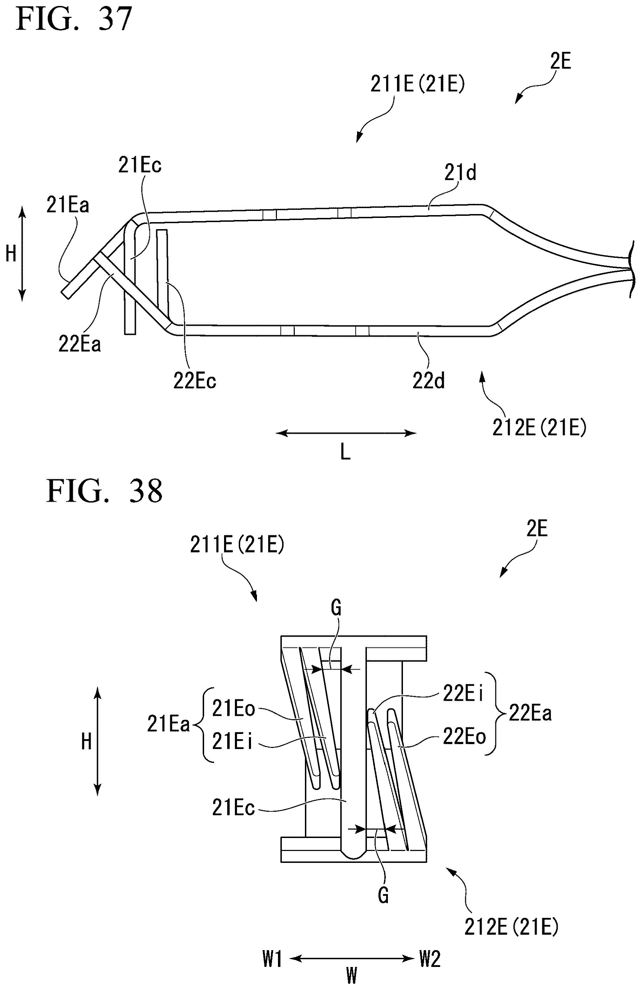

FIG. 36 is a view showing the clip 2E in an open state. FIG. 37 is a view showing the clip 2E in a closed state. The clip (clip arm) 2E is formed by bending a metal plate material at a central portion. The clip 2E has a pair of arms 21E that can be opened and closed toward the distal end side A1, and a proximal end portion 28 that connects the pair of arms 21E.

The pair of arms 21E have a first arm 211E and a second arm 212E that can be opened and closed in the opening and closing direction H. The first arm 211E and the second arm 212E are disposed on both sides of the central axis O1 of the clip unit 1E in the longitudinal direction A.

The first arm 211E has a first anchor 21Ea, a first claw 21Ec, a first arm main body 21d, and a first sliding portion 21e. The first anchor 21Ea has a first inner anchor 21Ei and a first outer anchor 21Eo.

The second arm 212E has a second anchor 22Ea, a second claw 22Ec, a second arm main body 22d, and a second sliding portion 22e. The second anchor 22Ea has a second inner anchor 22Ei and a second outer anchor 22Eo. The second arm 212E has a similar shape to the first arm 211E, and thus only the different portions will be described below.

FIG. 38 is a view showing the clip 2E in a closed state as viewed in the longitudinal direction L.

The first claw 21Ec and the first anchor 21Ea are spaced apart from each other in the width direction W. A gap G is formed between the first claw 21Ec and the first anchor 21Ea in the width direction W. For this reason, the first anchor 21Ea can easily puncture the mucous membrane.

The first claw 21Ec is provided at a distal end 21s of the first arm main body 21d and extends toward the closing side H1 in the opening and closing direction H. The first claw 21Ec is disposed in the center in the width direction W.

The first anchor 21Ea (first inner anchor 21Ei and first outer anchor 21 Eo) is provided at the distal end 21s of the first arm main body 21d and extends toward the closing side H1 in the opening and closing direction H. The first anchor 21Ea (first inner anchor 21Ei and first outer anchor 21Eo) is disposed on one side W1 in the width direction W. The first inner anchor 21Ei and the first outer anchor 21 Eo are arranged in the width direction W. A distal end of the first inner anchor 21Ei is formed in a sharp tapered shape, and thus it is possible to easily puncture the mucous membrane. A distal end of the first outer anchor 21 Eo is formed in a sharp tapered shape, and thus it is possible to easily puncture the mucous membrane.

The first anchor 21Ea (first inner anchor 21Ei and first outer anchor 21Eo) is disposed on the opening side H2 in the opening and closing direction H with respect to the first claw 21 Ec.

According to the clip unit 1E of the present embodiment, it is possible to suitably carry out the treatment of suturing a defective portion D. The first arm 211E and the second arm 212E each have a plurality of anchors, and thus it is possible to easily puncture the mucous membrane.

In the above, the fifth embodiment of the present invention has been described in detail with reference to the drawings, but the specific configuration is not limited to the embodiment, and a design change and the like within a range not departing from the gist of the present invention are also included. In addition, the constituent elements shown in the embodiment described above and a modification example which will be shown below can be appropriately combined and configured.

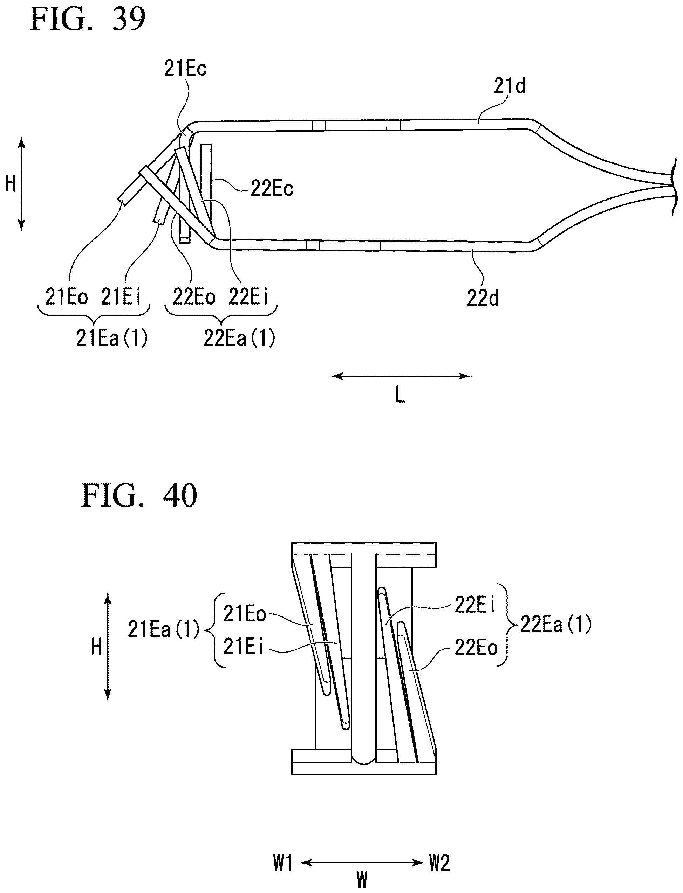

FIGS. 39 and 40 are views showing a first anchor 21Ea(1) which is a modification example of the first anchor 21Ea, and a second anchor 22Ea(1) which is a modification example of the second anchor 22Ea. A first inner anchor 21Ei and a first outer anchor 21 Eo of the first anchor 21Ea(1) have different angles with respect to the longitudinal direction L. The first inner anchor 21Ei and the first outer anchor 21 Eo have different angles at which they come into contact with the mucous membrane and thus can easily puncture the mucous membrane regardless of the angle of the clip 2E with respect to the mucous membrane.

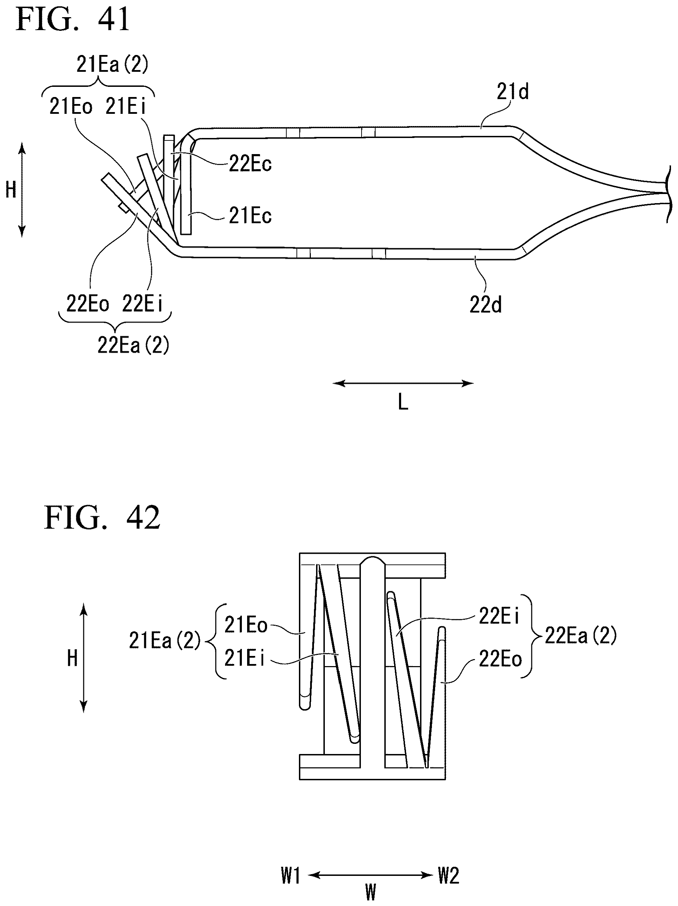

FIGS. 41 and 42 are views showing a first anchor 21Ea(2) which is a modification example of the first anchor 21Ea, and a second anchor 22Ea(2) which is a modification example of the second anchor 22Ea. A first inner anchor 21Ei and a first outer anchor 21 Eo of the first anchor 21Ea(2) have different angles with respect to the opening and closing direction H in addition to the longitudinal direction L. The first inner anchor 21Ei and the first outer anchor 21 Eo have different angles at which they come into contact with the mucous membrane and thus can easily puncture the mucous membrane regardless of the angle of the clip 2E with respect to the mucous membrane.

### (Sixth embodiment)

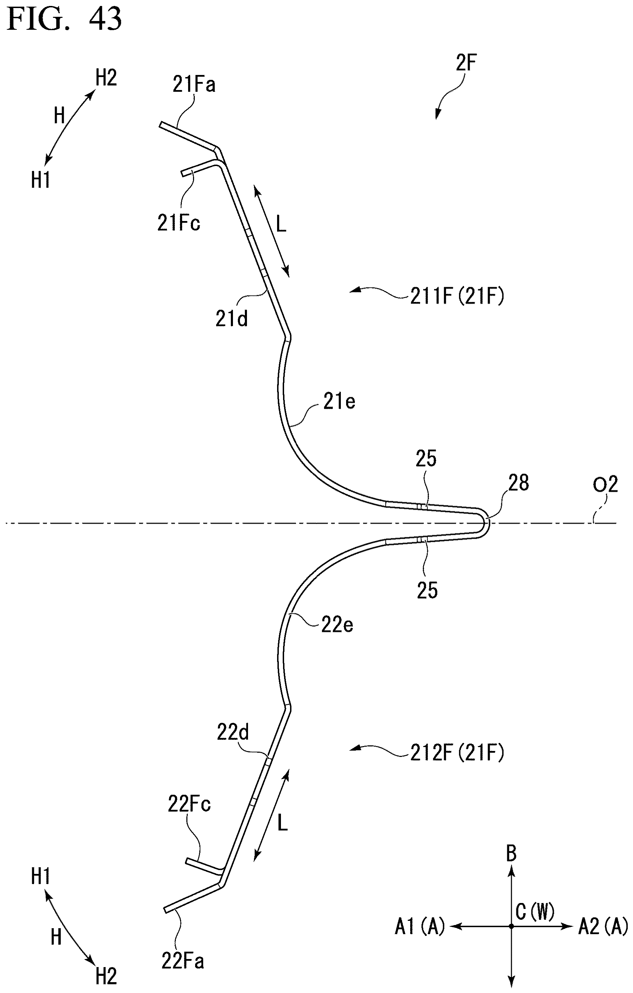

A clip unit 1F according to a sixth embodiment of the present invention will be described with reference to FIGS. 43 to 45. In the following description, the same constituent elements as those already described are designated by the same reference signs, and duplicate description will be omitted.

The clip unit 1F includes a clip 2F, the pressing member 3, and the connecting member 4. The clip unit 1F is loaded into the clip introducing device 200.

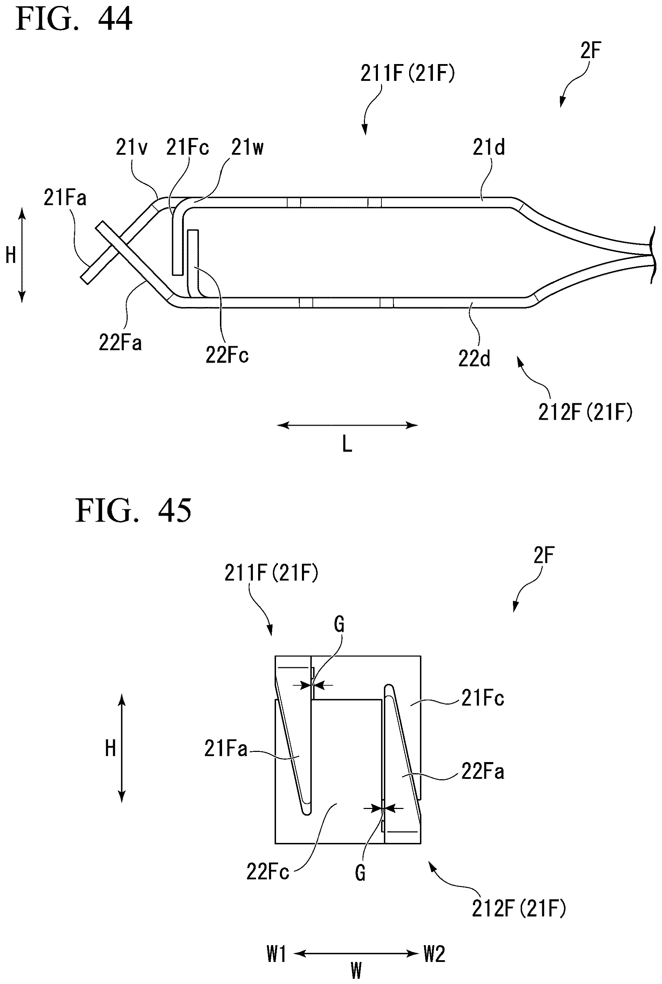

FIG. 43 is a view showing the clip 2F in an open state. FIG. 44 is a view showing the clip 2F in a closed state. The clip (clip arm) 2F is formed by bending a metal plate material at a central portion. The clip 2F has a pair of arms 21F that can be opened and closed toward the distal end side A1, and a proximal end portion 28 that connects the pair of arms 21F.

The pair of arms 21F have a first arm 211F and a second arm 212F that can be opened and closed in the opening and closing direction H. The first arm 211F and the second arm 212F are disposed on both sides of the central axis O1 of the clip unit 1F in the longitudinal direction A.

The first arm 211F has a first anchor 21Fa, a first claw 21Fc, a first arm main body 21d, and a first sliding portion 21e.

The second arm 212F has a second anchor 22Fa, a second claw 22Fc, a second arm main body 22d, and a second sliding portion 22e. The second arm 212F has a similar shape to the first arm 211F, and thus only the different portions will be described below.

FIG. 45 is a view showing the clip 2F in a closed state as viewed in the longitudinal direction L.

The first claw 21Fc and the first anchor 21Fa are spaced apart from each other in the width direction W. A gap G is formed between the first claw 21Fc and the first anchor 21Fa in the width direction W. For this reason, the first anchor 21Fa can easily puncture the mucous membrane.

The first anchor 21Fa is disposed on the opening side H2 in the opening and closing direction H with respect to the first claw 21Fc. The first claw 21Fc and the first anchor 21Fa extend from different portions when viewed in the width direction W as shown in FIG. 44. The first anchor 21Fa extends from a first connecting portion 21v. The first claw 21Fc extends from a second connecting portion 21w. The first connecting portion 21v is located on the distal end side in the longitudinal direction L with respect to the second connecting portion 21w.

According to the clip unit 1F of the present embodiment, it is possible to suitably carry out the treatment of suturing a defective portion D. Since the first claw 21Fc and the first anchor 21Fa are disposed at positions separated from each other in the longitudinal direction L, the first claw 21Fc does not get in the way when the first anchor 21Fa punctures the mucous membrane.

In the above, the sixth embodiment of the present invention has been described in detail with reference to the drawings, but the specific configuration is not limited to the embodiment, and a design change and the like within a range not departing from the gist of the present invention are also included. In addition, the constituent elements shown in the embodiment described above and a modification example which will be shown below can be appropriately combined and configured.

### (Seventh embodiment)

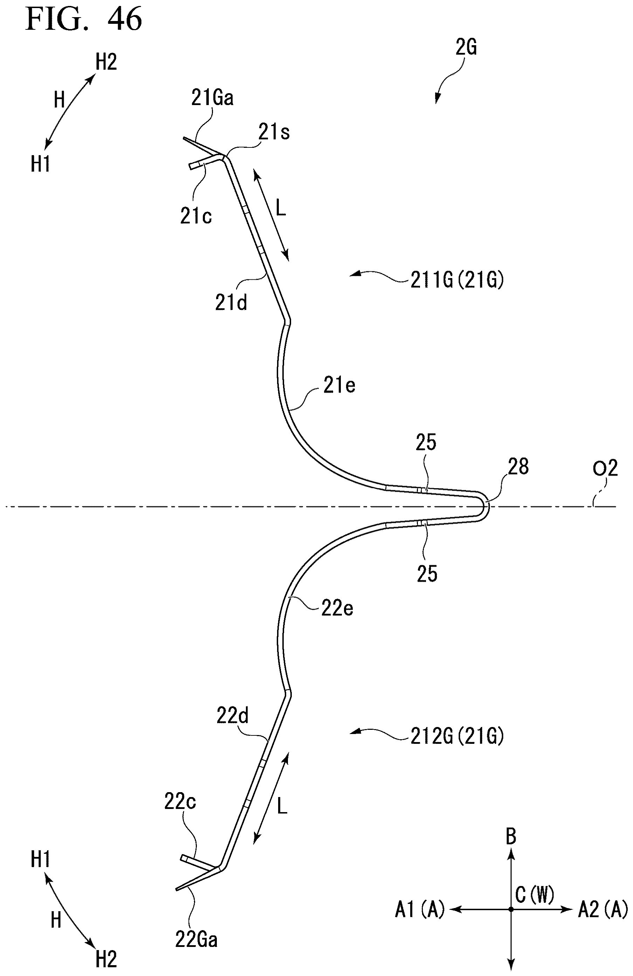

A clip unit 1G according to a seventh embodiment of the present invention will be described with reference to FIGS. 46 to 48. In the following description, the same constituent elements as those already described are designated by the same reference signs, and duplicate description will be omitted.

The clip unit 1G includes a clip 2G, the pressing member 3, and the connecting member 4. The clip unit 1G is loaded into the clip introducing device 200.

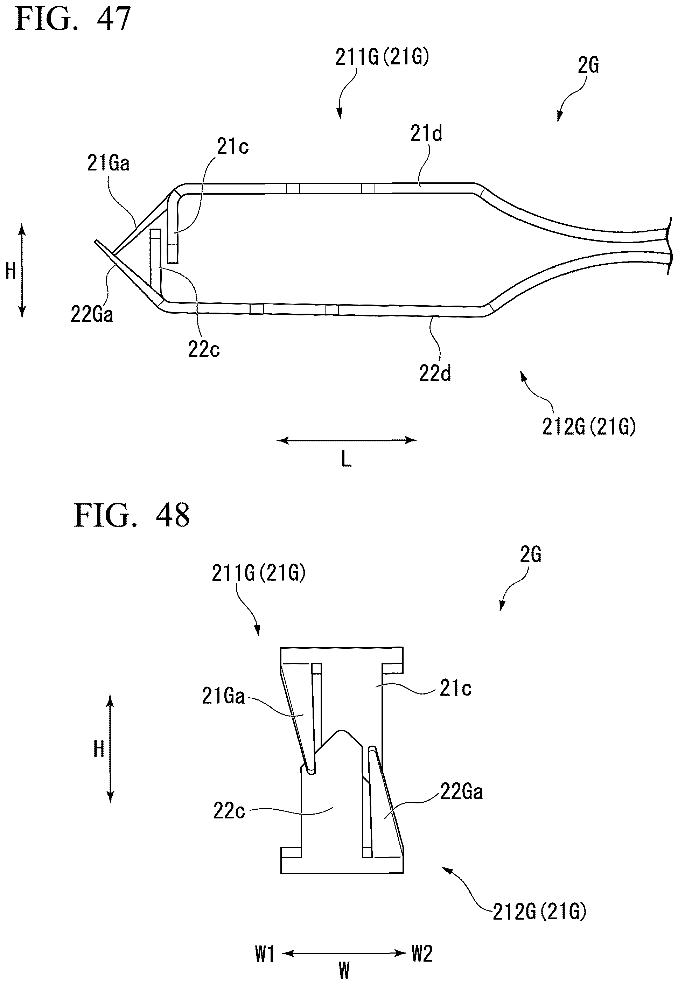

FIG. 46 is a view showing the clip 2G in an open state. FIG. 47 is a view showing the clip 2G in a closed state. The clip (clip arm) 2G is formed by bending a metal plate material at a central portion. The clip 2G has a pair of arms 21G that can be opened and closed toward the distal end side A1, and a proximal end portion 28 that connects the pair of arms 21G.

The pair of arms 21G have a first arm 211G and a second arm 212G that can be opened and closed in the opening and closing direction H. The first arm 211G and the second arm 212G are disposed on both sides of the central axis O1 of the clip unit 1G in the longitudinal direction A.

The first arm 211G has a first anchor 21Ga, a first claw 21c, a first arm main body 21d, and a first sliding portion 21e.

The second arm 212G has a second anchor 22Ga, a second claw 22c, a second arm main body 22d, and a second sliding portion 22e. The second arm 212G has a similar shape to the first arm 211G, and thus only the different portions will be described below.

FIG. 48 is a view showing the clip 2G in a closed state as viewed in the longitudinal direction L.

The first anchor 21Ga is provided at a distal end 21s of the first arm main body 21d and extends toward the closing side H1 in the opening and closing direction H. The first anchor 21Ga is disposed on one side W1 in the width direction W. A distal end of the first anchor 21Ga is formed in a sharp tapered shape, and thus it is possible to easily puncture the mucous membrane. The first anchor 21Ga is formed in the shape of a right triangle when viewed in the longitudinal direction L and is thinner toward the distal end side.

The first anchor 21Ga is disposed on the opening side H2 in the opening and closing direction H with respect to the first claw 21c.

According to the clip unit 1G of the present embodiment, it is possible to suitably carry out the treatment of suturing a defective portion D. The first anchor 21Ga is thinner toward the distal end side, and thus it is possible to easily puncture the mucous membrane.

In the above, the seventh embodiment of the present invention has been described in detail with reference to the drawings, but the specific configuration is not limited to the embodiment, and a design change and the like within a range not departing from the gist of the present invention are also included. In addition, the constituent elements shown in the embodiment described above and a modification example which will be shown below can be appropriately combined and configured.

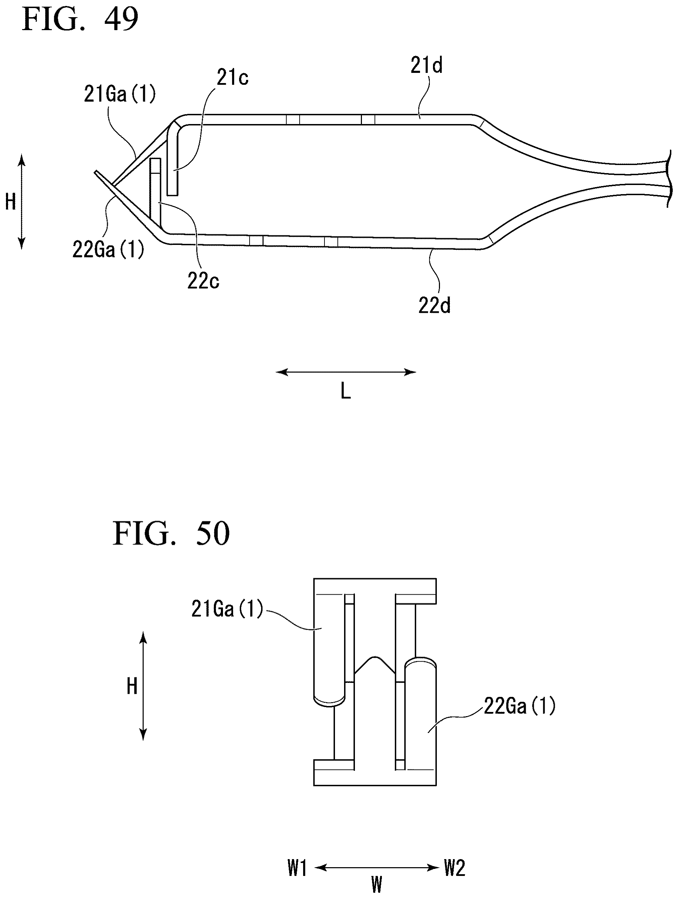

FIGS. 49 and 50 are views showing a first anchor 21Ga(1) which is a modification example of the first anchor 21 Ga, and a second anchor 22Ga(1) which is a modification example of the second anchor 22Ga. A distal end of the first anchor 21Ga(1) is not formed in a sharp tapered shape, but is thinner toward the distal end side, and thus it is possible to easily puncture the mucous membrane.

### (Eighth embodiment)

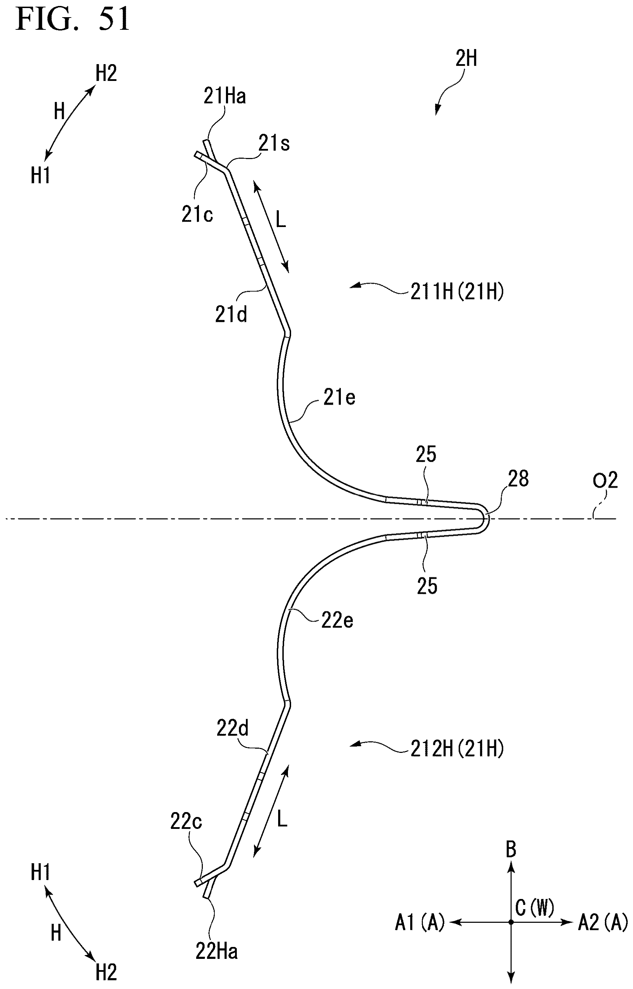

A clip unit 1H according to an eighth embodiment of the present invention will be described with reference to FIGS. 51 to 53. In the following description, the same constituent elements as those already described are designated by the same reference signs, and duplicate description will be omitted.

The clip unit 1H includes a clip 2H, the pressing member 3, and the connecting member 4. The clip unit 1H is loaded into the clip introducing device 200.

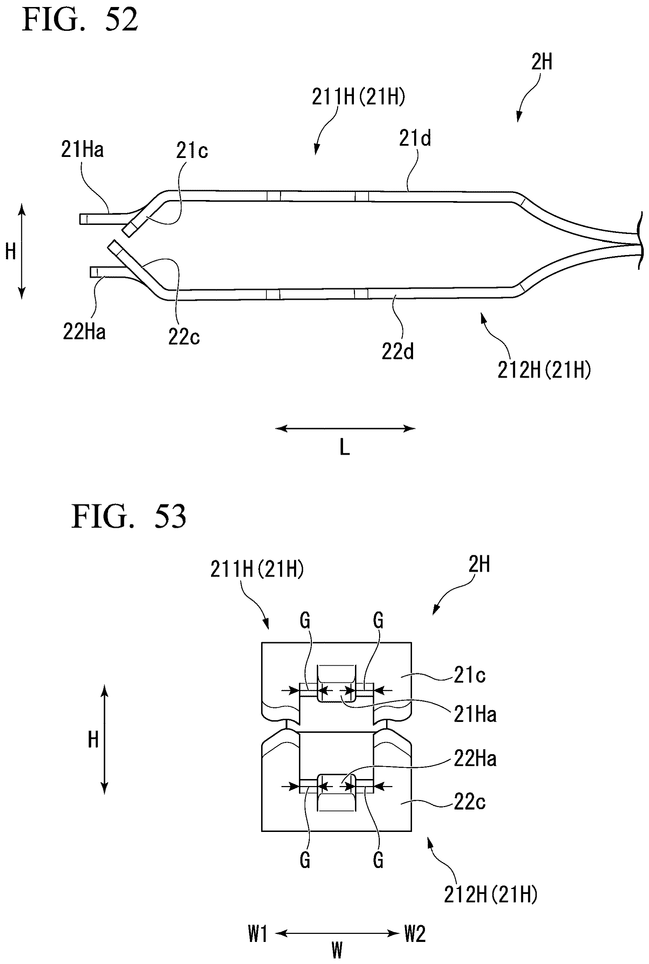

FIG. 51 is a view showing the clip 2H in an open state. FIG. 52 is a view showing the clip 2H in a closed state. The clip (clip arm) 2H is formed by bending a metal plate material at a central portion. The clip 2H has a pair of arms 21H that can be opened and closed toward the distal end side A1, and a proximal end portion 28 that connects the pair of arms 21H.

The pair of arms 21H have a first arm 211H and a second arm 212H that can be opened and closed in the opening and closing direction H. The first arm 211H and the second arm 212H are disposed on both sides of the central axis O1 of the clip unit 1H in the longitudinal direction A.

The first arm 211H has a first anchor 21Ha, a first claw 21c, a first arm main body 21d, and a first sliding portion 21e.

The second arm 212H has a second anchor 22Ha, a second claw 22c, a second arm main body 22d, and a second sliding portion 22e. The second arm 212H has a similar shape to the first arm 211H, and thus only the different portions will be described below.

FIG. 53 is a view showing the clip 2H in a closed state as viewed in the longitudinal direction L.

The first anchor 21Ha is provided on the first claw 21c and extends toward the closing side H1 in the opening and closing direction H. The first anchor 21Ha and the second anchor 22Ha are formed in a symmetrical shape with respect to a facing surface where the first arm 211H and the second arm 212H face each other.

The first anchor 21Ha is disposed on the opening side H2 in the opening and closing direction H with respect to the first claw 21c.

According to the clip unit 1H of the present embodiment, it is possible to suitably carry out the treatment of suturing a defective portion D. Since the first anchor 21Ha is disposed close to the mucous membrane, the first anchor 21Ha can easily puncture the mucous membrane even if it is short. Since the first anchor 21Ha is short, the first anchor 21Ha can easily puncture the mucous membrane without bending.

In the above, the eighth embodiment of the present invention has been described in detail with reference to the drawings, but the specific configuration is not limited to the embodiment, and a design change and the like within a range not departing from the gist of the present invention are also included. In addition, the constituent elements shown in the embodiment described above and a modification example which will be shown below can be appropriately combined and configured.

### (Ninth embodiment)

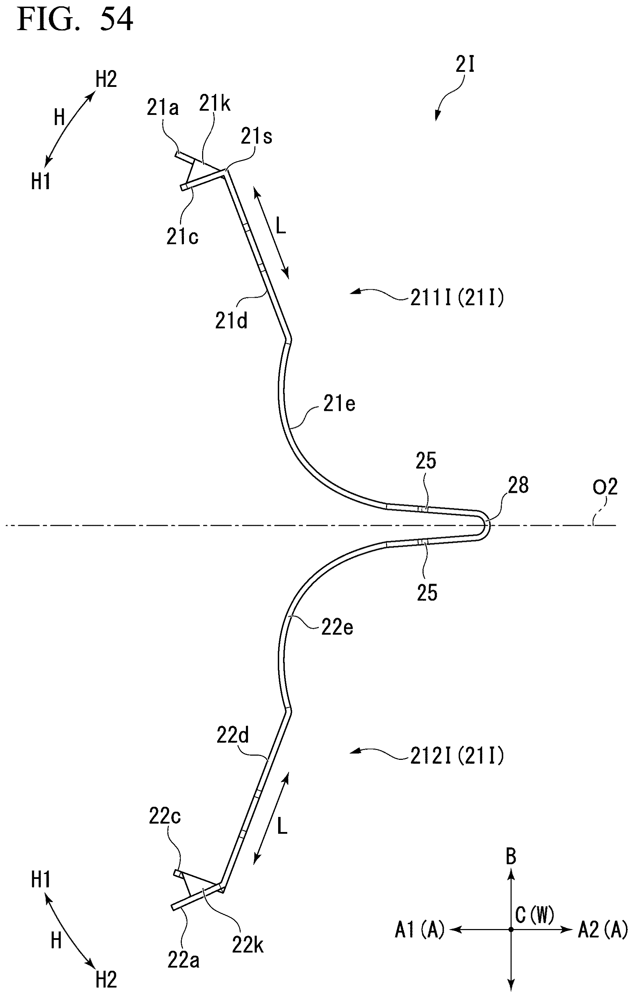

A clip unit 1I according to a ninth embodiment of the present invention will be described with reference to FIGS. 54 to 58. In the following description, the same constituent elements as those already described are designated by the same reference signs, and duplicate description will be omitted.

The clip unit 1I includes a clip 2I, the pressing member 3, and the connecting member 4. The clip unit 1I is loaded into the clip introducing device 200.

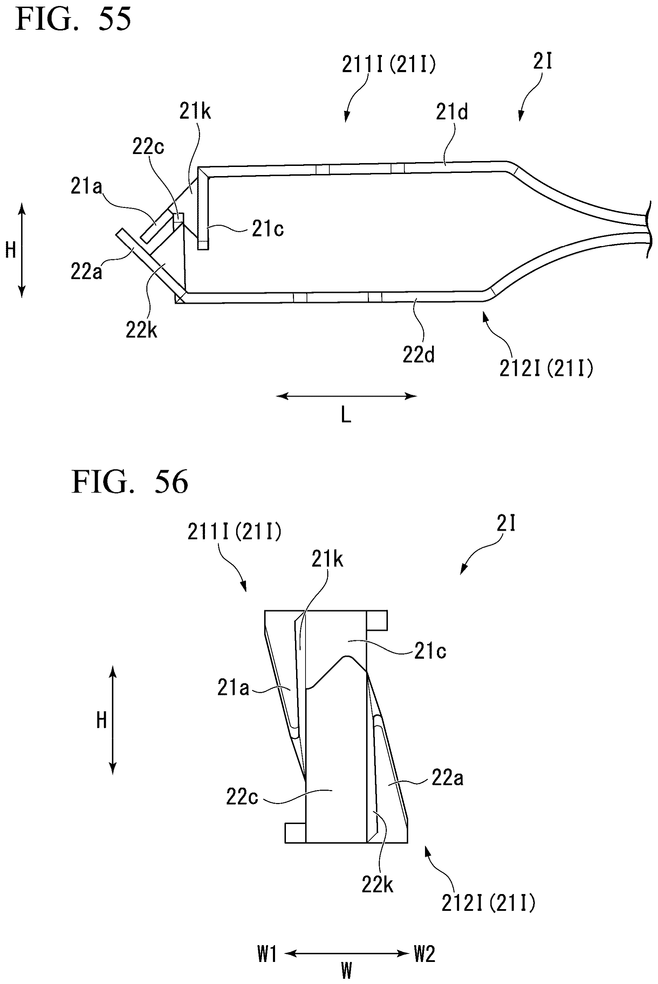

FIG. 54 is a view showing the clip 2I in an open state. FIG. 55 is a view showing the clip 2I in a closed state. The clip (clip arm) 2I is formed by bending a metal plate material at a central portion. The clip 2I has a pair of arms 21I that can be opened and closed toward the distal end side A1, and a proximal end portion 28 that connects the pair of arms 21I.

The pair of arms 21I have a first arm 211I and a second arm 212I that can be opened and closed in the opening and closing direction H. The first arm 211I and the second arm 212I are disposed on both sides of the central axis O1 of the clip unit 1I in the longitudinal direction A.

The first arm 211I has a first anchor 21a, a first claw 21c, a first fin portion 21k, a first arm main body 21d, and a first sliding portion 21e.

The second arm 212I has a second anchor 22a, a second claw 22c, a second fin portion 22k, a second arm main body 22d, and a second sliding portion 22e. The second arm 212I has a similar shape to the first arm 211I, and thus only the different portions will be described below.

FIG. 56 is a view showing the clip 2I in a closed state as viewed in the longitudinal direction L.

The first fin portion 21k is a fin-shaped member that connects the first anchor 21a and the first claw 21c. At least a part of a gap G formed between the first claw 21c and the first anchor 21Fa in the width direction W is filled with the first fin portion 21k.

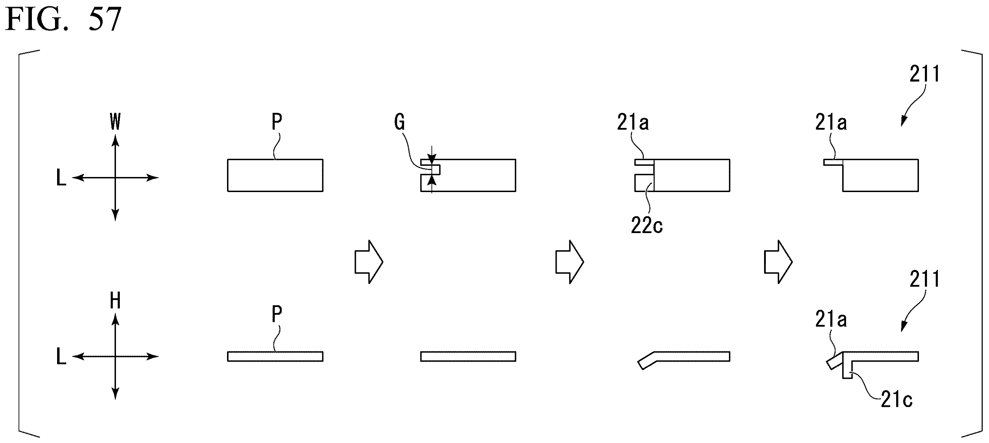

FIG. 57 is a view showing a manufacturing method of the first arm 211 of the first embodiment.

The manufacturing method of the first arm 211 has a step of punching a metal plate material P with a pressing machine to form the gap G, a step of forming the first anchor 21a through a primary bending process, and a step of forming the first claw 21c through a secondary bending process.

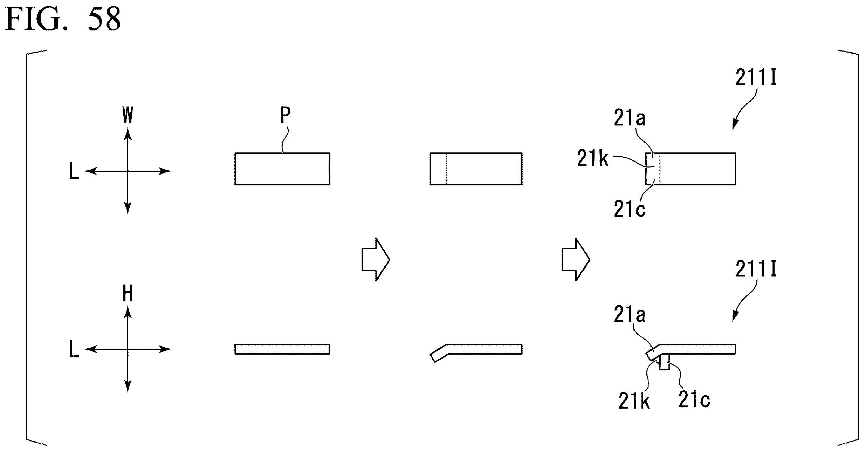

FIG. 58 is a view showing a manufacturing method of the first arm 211I of the ninth embodiment.

The manufacturing method of the first arm 2111 has a step of forming the first anchor 21a through a primary bending process on a metal plate material P, and a step of forming the first claw 21c through a secondary bending process. The manufacturing method of the first arm 211I does not require the punching step for forming the gap G. The first fin portion 21k is formed in the process of bending the metal plate material P in which the gap G is not formed.

According to the clip unit 1I of the present embodiment, it is possible to suitably carry out the treatment of suturing a defective portion D. The manufacturing method of the clip 2I does not require the punching step for forming the gap G. The first anchor 21a, the first claw 21c, and the first fin portion 21k can be easily formed through the bending process.

In the above, the ninth embodiment of the present invention has been described in detail with reference to the drawings, but the specific configuration is not limited to the embodiment, and a design change and the like within a range not departing from the gist of the present invention are also included. In addition, the constituent elements shown in the embodiment described above and a modification example which will be shown below can be appropriately combined and configured.

### INDUSTRIAL APPLICABILITY

The present invention can be applied to a clip unit or the like.

### REFERENCE SIGNS LIST

300 Clip device
200 Clip introducing device (applicator)
220 Sheath
221 Distal end tip
222 Distal end side coil
224 Proximal side coil
230 Operation wire (power transmission unit)
231 Arrowhead hook (connection section)
232 Wire
240 Operation section
1, 1B, 1C, 1D, 1E, 1F, 1G, 1H, 1I Clip unit
2, 2B, 2C, 2D, 2E, 2F, 2G, 2H, 2I Clip (clip arm)
21, 21B, 21C, 21D, 21E, 21F, 21G, 21H, 21I Pair of arms
211, 211B, 211C, 211D, 211E,211F, 211G, 211H, 2111 First Arm
212, 212B, 212C, 212D, 212E, 212F, 212G, 212H, 212I Second arm
25 Engaging portion
28 Proximal end portion
3 Pressing member (tubular member)
3A Pressing tube (second tubular member)
3B Pressing pipe (first tubular member)
30 Pressing tube main body
31 Projecting and retracting wing
32 Fastening member
38 Internal space
4 Connecting member
41 Distal end connecting portion
41b Breaking portion
42 Proximal end connecting portion
43 Connecting portion main body
43p Engaging convex portion
44 Connecting arm (pair of legs)
44e Insertion opening
44m Notch
44s Proximal end surface
45 Rod-shaped portion
A Longitudinal direction
A1 Distal end side (distal side)
A2 Proximal end side (proximal side)
B Left-right direction
C Up-and-down direction
L Longitudinal direction
H Opening and closing direction
W Width direction

## Claims

1. A clip unit comprising:
a clip having a first arm and a second arm that are able to be opened and closed in an opening and closing direction; and
a tubular member that is able to store at least a part of the clip,
wherein the first arm has
a first arm main body extending in a longitudinal direction along a longitudinal axis,
a first claw that is provided at a distal end of the first arm main body and extends toward a closing side in the opening and closing direction, and
a first anchor that is provided at the distal end of the first arm main body or the first claw and extends toward the closing side in the opening and closing direction,
wherein at least a distal end of the first anchor is disposed on an opening side in the opening and closing direction with respect to the first claw, and
wherein a gap is formed between the first claw and the first anchor in a width direction perpendicular to the longitudinal direction and the opening and closing direction.

2. The clip unit according to claim 1, wherein a length of the first anchor in the width direction is smaller than a length of the first claw in the width direction.

3. The clip unit according to claim 1, wherein a distal end of the first anchor is disposed on a distal end side with respect to a distal end of the first claw.

4. The clip unit according to claim 1, wherein a first angle formed between the first anchor and the first arm main body is greater than a second angle formed between the first claw and the first arm main body.

5. The clip unit according to claim 4, wherein the first angle is greater than or equal to 90 degrees and less than or equal to 135 degrees.

6. The clip unit according to claim 1,
wherein the first arm has a first auxiliary anchor, and
wherein the first anchor and the first auxiliary anchor are provided on both sides of the first claw in the width direction.

7. The clip unit according to claim 1,
wherein the first claw has a first upper claw and a first lower claw, and
wherein the first upper claw and the first lower claw are provided on both sides of the first anchor in the width direction.

8. The clip unit according to claim 1,
wherein the first anchor has a first inner anchor and a second outer anchor, and
wherein the first inner anchor and the second outer anchor are arranged in the width direction.

9. The clip unit according to claim 1, wherein a first connecting portion that connects the first anchor and the first arm main body is located on a distal end side in the longitudinal direction with respect to a second connecting portion that connects the first claw and the first arm main body.

10. The clip unit according to claim 1, wherein the first anchor is thinner toward a distal end side.

11. The clip unit according to claim 1, wherein the first anchor is provided on the first claw.

12. The clip unit according to claim 1, further comprising a first fin portion that fills at least a part of the gap formed between the first claw and the first anchor.

13. The clip unit according to claim 1,
wherein the second arm includes
a second arm main body extending in a longitudinal direction along a longitudinal axis,
a second claw that is provided at a distal end of the second arm main body and extends toward a closing side in the opening and closing direction, and
a second anchor that is provided at the distal end of the second arm main body or the second claw and extends toward the closing side in the opening and closing direction,
wherein at least a distal end of the second anchor is disposed on an opening side in the opening and closing direction with respect to the first claw, and
wherein the second claw and the second anchor are spaced apart from each other in a width direction perpendicular to the longitudinal direction and the opening and closing direction.

14. The clip unit according to claim 13, wherein a length of the first arm main body in the longitudinal direction is greater than a length of the second arm main body in the longitudinal direction.

15. The clip unit according to claim 13, wherein, when the first arm and the second arm are in a closed state, the first anchor and the second anchor are disposed to be spaced apart from each other when viewed in the longitudinal direction.

16. The clip unit according to claim 15, wherein, when the first arm and the second arm are in a closed state, the first anchor and the second anchor are disposed to overlap with each other at an overlapping position in the opening and closing direction when viewed in the longitudinal direction.

17. The clip unit according to claim 13, wherein, when the first arm and the second arm are in a closed state, the first claw and the second claw are disposed to overlap with each other when viewed in the longitudinal direction.

18. The clip unit according to claim 13, wherein, when the first arm and the second arm are in a closed state,
the first claw and the second claw are disposed at a center in the width direction,
the first anchor is disposed on one side in the width direction, and
the second anchor is disposed on the other side in the width direction.

19. The clip unit according to claim 13, wherein, when the first arm and the second arm are in a closed state, the first anchor and the second anchor are disposed to overlap with each other when viewed in the longitudinal direction.

20. The clip unit according to claim 13, wherein, when the first arm and the second arm are in a closed state, a gap is formed between the first anchor and the second anchor in the opening and closing direction.
